# EUROPEAN PATENT SPECIFICATION

(11) **EP 4 561 685 B1**
(45) Date of publication and mention of the grant of the patent: **23.09.2026**
(21) Application number: 23741570.8
(22) Date of filing: 20.06.2023
(51) Int. Cl.: A61B 5/353, A61N 1/365, A61B 5/352, A61B 5/00, A61B 5/363, A61N 1/372

(54) **ATRIOVENTRICULAR BLOCK DETECTION**
NACHWEIS VON ATRIOVENTRIKULÄRER BLOCKIERUNG
DÉTECTION DE BLOC ATRIOVENTRICULAIRE

(30) Priority: 29.07.2022 US 202263369861 P
(43) Date of publication of application: 04.06.2025
(73) Proprietor: Medtronic, Inc., Minneapolis, Minnesota 55432 (US)
(72) Inventor: CHAKRAVARTHY, Niranjan, Singapore 486056 (SG); KATRA, Rodolphe, Mounds View, Minnesota 55112 (US); CHO, Yong K., Mounds View, Minnesota 55112 (US)
(74) Representative: Zimmermann & Partner Patentanwälte mbB
(86) International application number: PCT/US2023/068754
(87) International publication number: WO 2024/026178

(56) References cited:
- US-A1- 2021 338 138
- US-A1- 2022 160 285
- US-A1- 2022 202 344

## Description

### FIELD

This disclosure generally relates to medical devices and, more particularly, to medical devices configured for cardiac monitoring.

### BACKGROUND

Atrioventricular (AV) block is an interruption of impulse transmission from the atria to the ventricles. AV block may be partial or complete. In addition, there are three types, or degrees, of AV block: first-degree, second-degree, and third-degree, with third-degree being the most severe. First-degree AV block is partial and occurs when the electrical signal (i.e., impulse) is delayed, but not disrupted, as the electrical signal moves between the atrium and the ventricles through the AV node. Second-degree AV block is partial and occurs when the electrical signal between the atria and ventricles is even more impaired than in a first-degree AV block. In a second-degree AV block, the impairment results in a failure to conduct an impulse, which causes a skipped beat. Second-degree AV block may be further classified as Mobitz I and Mobitz II. Third-degree AV block is complete and occurs when the signal between the atria and ventricles is completely blocked, and there is no communication and synchronization between the two. None of the signals from the upper chambers of the heart makes it to the lower chambers.

The most common cause of AV block is idiopathic fibrosis and sclerosis of the conduction system. Symptoms and treatment depend on degree of block, but treatment, when necessary, usually involves pacing. In patients with a high risk of AV block, the use of an implantable medical device (IMD), such as a pacemaker has shown to be beneficial. The pacemaker may deliver demand pacing in the absence of intrinsic ventricular activity, and restore the heart's normal rhythm. Some pacemakers deliver bradycardia pacing, cardiac resynchronization therapy (CRT), and/or other forms of pacing.

Other types of medical devices may be used for diagnostic purposes. For instance, an implanted or non-implanted medical device may monitor a patient's heart. A user, such as a physician, may review data generated by the medical device for occurrences of cardiac arrhythmias, e.g., atrial or ventricular tachyarrhythmia, or asystole.

US 2022/160285 A1 relates to non-invasive cardiac monitor and methods of using recorded cardiac data to infer a physiological characteristic of a patient.

US 2022/202344 A1 relates to an apparatus and method for electrocardiogram (ECG) signal analysis and heart block detection.

US 2021/338138 A1 relates to a visualization of arrhythmia detection by machine learning.

### SUMMARY

In accordance with the techniques of the disclosure, a medical device system is set forth herein that detects and classifies AV block in a patient. For example, at least one computing device (e.g., an external device, a computing system, etc.) receives cardiac electrogram data of a patient sensed by an IMD. The IMD may have stored the cardiac electrogram data based on detecting an arrhythmia episode, e.g., bradycardia or pause, in the cardiac electrogram data. The computing device determines a R-R interval and whether a P-wave is detectable in the cardiac electrogram data. Based on determining that the P-wave is detectable, the computing device determines a P-wave location in the cardiac electrogram data and a P-R interval. The computing device determines whether an episode of AV block has occurred within the cardiac electrogram data. Responsive to determining that the episode of AV block has occurred, the computing device outputs a report. The report may include an indication that the episode of AV block has occurred in the patient and at least one of the R-R intervals or the P-R intervals that coincide with the episode of AV block.

The techniques of the disclosure may provide one or more improvements to the field of cardiac arrhythmia detection and classification. For example, the techniques described herein may improve the accuracy of the detection of AV block in a patient. The techniques may allow a computing device to detect AV blocks present in episodes of other arrhythmias detected by an IMD. The IMD may detect the other arrhythmias with greater sensitivity and specificity than AV block, and the computing device may detect AV block in episodes of the other arrhythmias with greater sensitivity and specificity than detection of AV block by the IMD.

Furthermore, the techniques of the disclosure may help a clinician interpret and/or explain determinations of AV block by the medical device system, increasing confidence in the ability of the medical device system to correctly detect AV block. In general, explainability may be important for the application of medical technology. For example, machine learning systems (e.g., machine learning (ML) models) that are not only effective (e.g., at accurately diagnosing and analyze cardiac electrogram data, thereby reducing the workload of a clinician) but are also transparent, interpretable, and explainable for a human expert may enhance trust of medical professionals and resolve potential medical, ethical, and/or societal concerns. For example, unlike conventional techniques, the techniques of the disclosure may determine that AV block has occurred during an episode of bradycardia or pause, which may be particularly helpful to a clinician interpret and/or explain determinations of AV block by the medical device system, increasing confidence in the ability of the medical device system to correctly detect AV block. The techniques enabling processing circuitry to identify AV block, e.g., using machine learning models, may greatly benefit clinicians with respect to time saving in the identification of AV block. Thus, by helping clinicians understand the decision process of the analyses described herein, the techniques of this disclosure may promote the acceptance and use of highly sophisticated and effective medical technology by the medical community and the general public.

In some examples, a method comprises: receiving, by a computing device and from a medical device, cardiac electrogram data of a patient sensed by the medical device for an episode recorded by the medical device; identifying, by the computing device, a plurality of heartbeats within the cardiac electrogram data for the episode; for each of the plurality of heartbeats: determining, by the computing device, a R-R interval; determining, by the computing device, whether a P-wave is detectable in the cardiac electrogram data; and responsive to determining that the P-wave is detectable, determining, by the computing device, a P-wave location in the cardiac electrogram data and a P-R interval; determining, by the computing device, whether atrioventricular block has occurred during the episode; and responsive to determining that atrioventricular block has occurred during the episode, outputting, by the computing device, a report comprising: an indication that atrioventricular block has occurred in the patient; and at least one of the R-R intervals or the P-R intervals that coincide with the atrioventricular block that has occurred during the episode.

In some examples, a system comprises: a medical device configured to sense cardiac electrogram data of a patient; and a computing device comprising processing circuitry configured to: receive, from the medical device, the cardiac electrogram data of the patient for an episode recorded by the medical device; identify a plurality of heartbeats within the cardiac electrogram data for the episode; for each of the plurality of heartbeats: determine a R-R interval; determine whether a P-wave is detectable in the cardiac electrogram data; and responsive to determining that the P-wave is detectable, determine a P-wave location in the cardiac electrogram data and a P-R interval; determine whether atrioventricular block has occurred during the episode; and responsive to determining that atrioventricular block has occurred during the episode, output a report comprising: an indication that atrioventricular block has occurred in the patient; and at least one of the R-R intervals or the P-R intervals that coincide with the atrioventricular block that has occurred during the episode.

This summary is intended to provide an overview of the subject matter described in this disclosure. It is not intended to provide an exclusive or exhaustive explanation of the apparatus and methods described in detail within the accompanying drawings and description below. Further details of one or more examples are set forth in the accompanying drawings and the description below. The invention is defined by independent claim 1. Embodiments of the invention are defined in the dependent claims.

### BRIEF DESCRIPTION OF DRAWINGS

FIG. 1 is a conceptual drawing illustrating an example of a medical device system configured to detect and/or classify AV block in conjunction with a patient in accordance with the techniques of the disclosure.
FIG. 2 is a block diagram illustrating an example of the implantable medical device of FIG. 1.
FIG. 3 is a block diagram illustrating another example of the implantable medical device of FIG. 1.
FIG. 4 is a block diagram illustrating an example computing device that operates in accordance with one or more techniques of the present disclosure.
FIG. 5 is a flowchart illustrating an example operation in accordance with the techniques of the disclosure.
FIG. 6 is a flowchart illustrating an example operation in accordance with the techniques of the disclosure.
FIG. 7 is a chart illustrating an example electrocardiogram obtained from the patient of FIG. 1.
FIG. 8 is a flowchart illustrating an example operation in accordance with the techniques of the disclosure.
FIG. 9 is a flowchart illustrating an example operation in accordance with the techniques of the disclosure.
FIG. 10 is a flowchart illustrating an example operation in accordance with the techniques of the disclosure.
FIG. 11 is a flowchart illustrating an example operation in accordance with the techniques of the disclosure.

Like reference characters refer to like elements throughout the figures and description.

### DETAILED DESCRIPTION

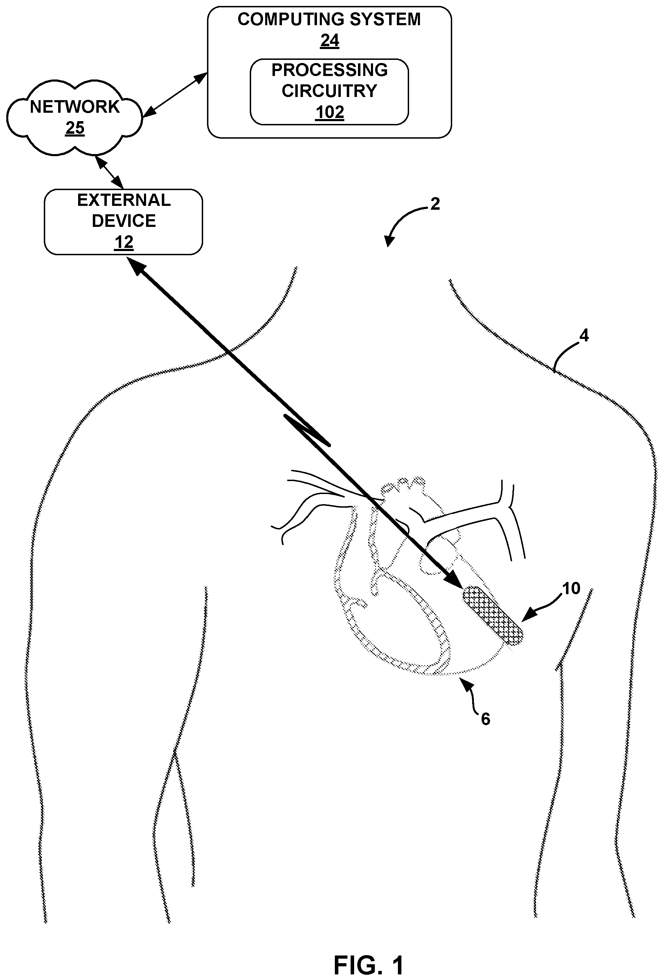

FIG. 1 illustrates the environment of an example medical device system 2 in conjunction with a patient 4 and a heart 6, in accordance with an apparatus and method of certain examples described herein. The example techniques may be used with an IMD 10, which may be leadless and in wireless communication with external device 12, as illustrated in FIG. 1. In some examples, IMD 10 may be coupled to one or more leads. In some examples, IMD 10 may be implanted outside of a thoracic cavity of patient 4 (e.g., subcutaneously in the pectoral location illustrated in FIG. 1). IMD 10 may be positioned near the sternum near and/or just below the level of heart 6.

In some examples, IMD 10 may take the form of a Reveal LINQ^{™} Insertable Cardiac Monitor (ICM), available from Medtronic plc, of Dublin, Ireland. External device 12 may be a computing device configured for use in settings such as a home, clinic, or hospital, and may further be configured to communicate with IMD 10 via wireless telemetry. For example, external device 12 may be coupled to computing system 24 via network 25. Computing system 24 may include a remote patient monitoring system, such as Carelink^{®}, available from Medtronic plc, of Dublin, Ireland. External device 12 may, in some examples, comprise a communication device such as a programmer, an external monitor, or a mobile device, such as a mobile phone, a "smart" phone, a laptop, a tablet computer, a personal digital assistant (PDA), etc.

In some examples, the techniques described herein may be used with an external medical device in addition to, or instead of IMD 10. In some examples, the external medical device is a wearable electronic device, such as the SEEQ^{™} Mobile Cardiac Telemetry (MCT) system formerly available from Medtronic plc, of Dublin, Ireland, or another type of wearable "smart" electronic apparel, such as a "smart" watch, "smart" patch, or "smart" glasses. Such an external medical device may be positioned externally to patient 4 (e.g., positioned on the skin of patient 4) and may carry out any or all of the functions described herein with respect to IMD 10.

In some examples, a user, such as a physician, technician, surgeon, electro-physiologist, or other clinician, may interact with external device 12 to retrieve physiological or diagnostic information from IMD 10. In some examples, a user, such as patient 4 or a clinician as described above, may also interact with external device 12 to program IMD 10, e.g., select or adjust values for operational parameters of IMD 10. In some examples, external device 12 acts as an access point to facilitate communication with IMD 10 via network 25, e.g., by computing system 24. Computing system 24 may comprise computing devices configured to allow a user to interact with IMD 10 via network 25.

In some examples, computing system 24 includes at least one of a handheld computing device, computer workstation, server or other networked computing device, smartphone, tablet, or external programmer that includes a user interface for presenting information to and receiving input from a user. In some examples, computing system 24 may include one or more devices that implement a machine learning system, such as neural network, a deep learning system, or other type of predictive analytics system. A user, such as a physician, technician, surgeon, electro-physiologist, or other clinician, may interact with computing system 24 to retrieve physiological or diagnostic information from IMD 10. A user may also interact with computing system 24 to program IMD 10, e.g., select values for operational parameters of IMD 10. Computing system 24 may include a processor configured to evaluate EGM and/or other sensed signals transmitted from IMD 10 to computing system 24.

Network 25 may include one or more computing devices (not shown), such as one or more non-edge switches, routers, hubs, gateways, security devices such as firewalls, intrusion detection, and/or intrusion prevention devices, servers, computer terminals, laptops, printers, databases, wireless mobile devices such as cellular phones or personal digital assistants, wireless access points, bridges, cable modems, application accelerators, or other network devices. Network 25 may include one or more networks administered by service providers, and may thus form part of a large-scale public network infrastructure, e.g., the Internet. Network 25 may provide computing devices, such as computing system 24 and IMD 10, access to the Internet, and may provide a communication framework that allows the computing devices to communicate with one another. In some examples, network 25 may be a private network that provides a communication framework that allows computing system 24, IMD 10, and/or external device 12 to communicate with one another but isolates one or more of computing system 24, IMD 10, or external device 12 from devices external to network 25 for security purposes. In some examples, the communications between computing system 24, IMD 10, and external device 12 are encrypted.

External device 12 and computing system 24 may communicate via wireless communication over network 25 using any techniques known in the art. In some examples, computing system 24 is a remote device that communicates with external device 12 via an intermediary device located in network 25, such as a local access point, wireless router, or gateway. While in the example of FIG. 1, external device 12 and computing system 24 communicate over network 25, in some examples, external device 12 and computing system 24 communicate with one another directly. Examples of communication techniques may include, for example, communication according to the Bluetooth ^{®} or BLE protocols. Other communication techniques are also contemplated. Computing system 24 may also communicate with one or more other external devices using a number of known communication techniques, both wired and wireless.

In any such examples, processing circuitry of medical device system 2, e.g., IMD 10, may transmit patient data, including cardiac electrogram data, for patient 4 to a computing device (e.g., external device 12 or computing system 24). In some examples, processing circuitry of medical device system 2 may transmit a determination that patient 4 is undergoing or has undergone an episode of cardiac arrhythmia such as an episode of bradycardia, tachycardia, atrial fibrillation, ventricular fibrillation, or AV Block.

External device 12 may be a computing device (e.g., used in a home, ambulatory, clinic, or hospital setting) to communicate with IMD 10 via wireless telemetry. External device 12 may include or be coupled to a remote patient monitoring system, such as Carelink^{®}, available from Medtronic plc, of Dublin, Ireland. In some examples, external device 12 may receive data, alerts, patient physiological information, or other information from IMD 10.

External device 12 may be used to program commands or operating parameters into IMD 10 for controlling its functioning (e.g., when configured as a programmer for IMD 10). In some examples, external device 12 may be used to interrogate IMD 10 to retrieve data, including device operational data as well as physiological data accumulated in IMD memory. Such interrogation may occur automatically according to a schedule and/or may occur in response to a remote or local user command. Programmers, external monitors, and consumer devices are examples of external devices 12 that may be used to interrogate IMD 10. Examples of communication techniques used by IMD 10 and external device 12 include radiofrequency (RF) telemetry, which may be an RF link established via Bluetooth, WiFi, or medical implant communication service (MICS). In some examples, external device 12 may include a user interface configured to allow patient 4, a clinician, or another user to remotely interact with IMD 10. In some such examples, external device 12, and/or any other device of medical device system 2, may be a wearable device, (e.g., in the form of a watch, necklace, or other wearable item).

Medical device system 2 is an example of a medical device system configured to perform cardiac arrhythmia detection, verification, and reporting. In accordance with the techniques of the disclosure, medical device system 2 detects and classifies cardiac arrhythmias in patient 4. Examples of the one or more other implanted or external devices may include an implanted, multi-channel cardiac pacemaker, ICD, IPG, leadless (e.g., intracardiac) pacemaker, extravascular pacemaker and/or ICD, or other IMD or combination of such IMDs configured to deliver CRT to heart 6, an external monitor, an external therapy delivery device such as an external pacing or electrical stimulation device, or a drug pump.

Communication circuitry of each of the devices of medical device system 2 (e.g., IMD 10 and external device 12) may enable the devices to communicate with one another. In addition, although one or more sensors (e.g., electrodes) are described herein as being positioned on a housing of IMD 10, in other examples, such sensors may be positioned on a housing of another device implanted in or external to patient 4. In such examples, one or more of the other devices may include processing circuitry configured to receive signals from the electrodes or other sensors on the respective devices and/or communication circuitry configured to transmit the signals from the electrodes or other sensors to another device (e.g., external device 12) or server.

AV block is partial or complete interruption of impulse transmission from the atria to the ventricles. Diagnosis is typically by electrocardiography. For example, a clinician may evaluate AV conduction of a patient by assessing the relationship between the P waves (i.e., atrial depolarizations) and QRS complexes (i.e., ventricular depolarizations). In a healthy patient, each P wave is followed by a QRS complex. The PR interval is normally between about 120 to about 200 milliseconds (ms).

A first-degree AV block is indicated on an ECG by a prolonged PR interval (e.g., a PR interval greater than 200 ms). A second-degree AV block is indicated on an ECG by varying failure of conduction through the AV node such that some P waves are not followed by corresponding QRS complexes. That is, unlike with first-degree AV block, a 1:1 P-wave-to-QRS-complex ratio is not maintained. A third-degree AV block is indicated on an ECG by P-waves that are completely unrelated to the QRS complexes, meaning the P-waves occur at one rate and the QRS complexes at another, a phenomenon sometimes referred to as "AV dissociation."

In accordance with the techniques of the disclosure, medical device system 2 detects cardiac arrhythmia, such as an episode of AV block, in patient 4. IMD 10 (or another medical device, which may or may not be implantable), may be configured to sense cardiac electrogram data of patient 4. The cardiac electrogram data may be for or otherwise associated with an episode recorded by IMD 10. In some examples, IMD 10 may record the episode in response to one or more R-R intervals (e.g., the time elapsed between two successive R-wave locations) in the cardiac electrogram data satisfying a duration condition. In some examples, a R-R interval may satisfy the duration condition when the R-R interval is equal to or greater than a threshold value. For example, if a threshold value is 1.5 seconds and at least one of the R-R intervals is equal to or greater than 1.5 seconds, IMD 10 may record the episode. In some examples, the threshold value may be a percentage (e.g., 130%) of the mean R-R interval of two or more heartbeats (e.g., 4 successive heartbeats) such that detection of a long-RR beat is based on deviation from a mean heart rate. In some examples, IMD 10 may record the episode if a R-R interval satisfies the duration condition and the R-R interval is not associated with a heartbeat that is a compensatory beat after a premature ventricular contraction (PVC). In some examples, IMD 10 may record the episode if N consecutive R-R intervals, or M of N consecutive R-R intervals satisfy a duration threshold.

In some examples, IMD 10 may record the episode based on heart rate variability (HRV) or R-R variability. For instance, if IMD 10 determines, based on the cardiac electrogram data, that the patient is experiencing relatively high HRV, then IMD 10 may record the episode. In some examples, IMD 10 may record the episode in response to an input from a patient that the patient provides (via, e.g., a patient activated event recorder) when the patient experiences symptoms.

Computing system 24, which may include one or more computing devices, may receive the cardiac electrogram data for an episode from IMD 10. The episode may be a bradycardia, a pause (asystole), an atrial fibrillation, an atrial tachycardia, a tachycardia, or a patient-activated episode, etc. Processing circuitry 102 of computing system 24 may be configured to identify a plurality of heartbeats within the cardiac electrogram data for the episode.

Processing circuitry 102 may analyze each heartbeat of the plurality of heartbeats. For example, processing circuitry 102 may determine a R-R interval. Processing circuitry 102 may further determine whether a P-wave is detectable in the cardiac electrogram data. Processing circuitry 102 may determine whether a P-wave is detectable using known P-wave detection techniques. An example P-wave detection technique may include analyzing the cardiac electrogram data for small positive deflections from the isoelectric baseline that (in a normal patient) precede corresponding QRS complexes. As discussed above, P-R intervals are often used to understand the type of AV block. Thus, confirming P-wave presence may be important, especially for devices whose signal acquisition and/or implant location may make the P-waves undetectable or otherwise "invisible."

Processing circuitry 102 may determine a P-wave location in the cardiac electrogram data and a P-R interval (e.g., the time elapsed between the P-wave location and R-wave location of a single heartbeat). In some examples, responsive to determining that one or more P-waves are not detectable, processing circuitry 102 may output a report comprising an indication that whether AV block has occurred during the episode is indeterminate.

Processing circuitry 102 may determine whether AV block has occurred during the episode. For instance, processing circuitry 102 may determine whether AV block has occurred based on the determined P-R intervals. For example, if processing circuitry 102 determines that one of the P-R intervals exceeds 200 ms (or another appropriate threshold value) but the 1:1 P-wave-to-QRS-complex ratio is maintained, processing circuitry 102 may determine that a first-degree AV block has occurred during the episode recorded by IMD 10. In another example, if processing circuitry 102 determines that the P-R intervals indicate that a 1:1 P-wave-to-QRS-complex ratio is not maintained, processing circuitry 102 may determine that a second-degree AV block has occurred. In the same example, if the P-R intervals become progressively longer, processing circuitry 102 may determine that a type 1 second-degree AV block (i.e., Mobitz I) has occurred. Otherwise, processing circuitry 102 may determine that a type 2 second-degree AV block (i.e., Mobitz II) has occurred. In yet another example, if processing circuitry 102 determines, based on the P-R intervals and R-R intervals, that the P-waves occur at one rate and the QRS complexes at another, processing circuitry 102 may determine that a third-degree AV block has occurred.

In some examples, processing circuitry 102 may exclude specific heartbeats of the episode when determining whether AV block has occurred based on P-R intervals. For example, processing circuitry 102 may exclude P-R intervals for heartbeats that adjacently follow a premature ventricular contraction heartbeat such that processing circuitry 102 cannot determine whether AV block has occurred based on P-R intervals for heartbeats that adjacently follow a premature ventricular contraction heartbeat. In another example, processing circuitry 102 may determine whether the R-R interval of each heartbeat satisfies an R-R interval threshold. Processing circuitry may exclude P-R intervals for heartbeats for which the R-R interval threshold is not satisfied.

In some examples, processing circuitry 102 may execute a machine learning system to determine whether a cardiac arrhythmia has occurred during the episode. For instance, processing circuitry 102 (or other components of medical device system 2) may use both feature delineation and machine learning to detect and classify cardiac arrhythmia (e.g., as AV block) in patient 4. For example, processing circuitry 102 may obtain, via feature-based delineation of the cardiac electrogram data, a first classification of arrhythmia in patient 4. In some examples, the feature-based delineation of the cardiac electrogram data to determine the first classification of arrhythmia in patient 4 is performed by any one of IMD 10, external device 12, or computing system 24. The machine learning system of computing system 24 may apply a machine learning model to the received cardiac electrogram data to obtain a second classification of arrhythmia in patient 4.

As one example, computing system 24 uses the first and second classifications to determine whether an episode of arrhythmia has occurred in patient 4. As another example, computing system 24 uses the second classification of arrhythmia obtained from the machine learning system to verify the first classification of arrhythmia in patient 4 obtained from the feature-based delineation. In some examples, processing circuitry 102 may execute the machine learning system to determine whether AV block has occurred within a particular episode without using feature delineation to make an additional determination of whether AV block occurred within the episode. In some of these examples, processing circuitry 102 may apply feature delineation to the episode, and provide feature data with a determination or classification made by the machine learning system to improve a user's understanding of and confidence in the determination or classification.

In one example, the machine learning model is a deep-learning model. In general, processing circuitry 102 may apply any one or more of models, algorithms, decision trees, and/or thresholds to determine whether an episode includes an AV block. Example machine learning techniques that may be employed to generate rules 250 can include various learning styles, such as supervised learning, unsupervised learning, and semi-supervised learning. Example types of algorithms include Bayesian algorithms, Clustering algorithms, decision-tree algorithms, regularization algorithms, regression algorithms, instance-based algorithms, artificial neural network algorithms, deep learning algorithms, dimensionality reduction algorithms and the like. Various examples of specific algorithms include Bayesian Linear Regression, Boosted Decision Tree Regression, and Neural Network Regression, Back Propagation Neural Networks, Convolution Neural Networks (CNN), Long Short Term Networks (LSTM), the Apriori algorithm, K-Means Clustering, k-Nearest Neighbour (kNN), Learning Vector Quantization (LVQ), Self-Organizing Map (SOM), Locally Weighted Learning (LWL), Ridge Regression, Least Absolute Shrinkage and Selection Operator (LASSO), Elastic Net, and Least-Angle Regression (LARS), Principal Component Analysis (PCA) and Principal Component Regression (PCR).

In some examples, medical device system 2 may classify arrhythmia according to an arrhythmia dictionary. As described in more detail below, computing system 24 may determine, via feature-based delineation of the cardiac electrogram data of patient 4, that AV block has potentially occurred. The machine learning system may apply a machine learning model to compare cardiac features coinciding with the potential occurrence of AV block with cardiac features of past occurrences of AV block so as to classify the arrhythmia as an arrhythmia of a particular type.

In examples where processing circuitry 102 does not determine whether AV block has occurred based on P-R intervals for specific heartbeats, processing circuitry 102 may still apply a machine learning system to the specific heartbeats (and other heartbeats) to determine whether AV block has occurred. For example, the machine learning system of computing system 24 may apply a machine learning model to heartbeats that adjacently follow a premature ventricular contraction heartbeat to determine whether AV block has occurred. Similarly, the machine learning system of computing system 24 may apply a machine learning model to heartbeats for which the R-R interval threshold is not satisfied to determine whether AV block has occurred.

In any case, responsive to determining that AV block has occurred during the episode recorded by IMD 10 in patient 4, computing system 24 may output a report including an indication that AV block has occurred in patient 4. The report may further include at least one of the R-R intervals or the P-R intervals that coincide with the AV block that has occurred during the episode. For example, if a machine learning system executed by processing circuitry 102 determines that AV block has occurred, the report may include an indication that AV block has occurred and include at least one of the R-R intervals or the P-R intervals that coincide with the AV block. In this way, the techniques of this disclosure may provide a clinician with information that explains the determination of AV block, thereby potentially increasing confidence in the accuracy of the diagnosis.

In examples, the report may exclude some information. For example, the report may exclude P-R intervals for specific heartbeats, such as heartbeats that adjacently follow a premature ventricular contraction heartbeat. In some examples, computing system 24 may determine, for each of the plurality of heartbeats, whether the R-R interval satisfies an R-R interval threshold. In such examples, the report may exclude P-R intervals for heartbeats for which the R-R interval threshold is not satisfied.

It should be understood that processing circuitry of medical device system 2 other than processing circuitry 102 of computing system 24 may perform at least a part of the techniques of this disclosure. For example, processing circuitry of IMD 10, external device 12, etc., may execute one or more algorithms, such as a machine learning model, to detect AV block.

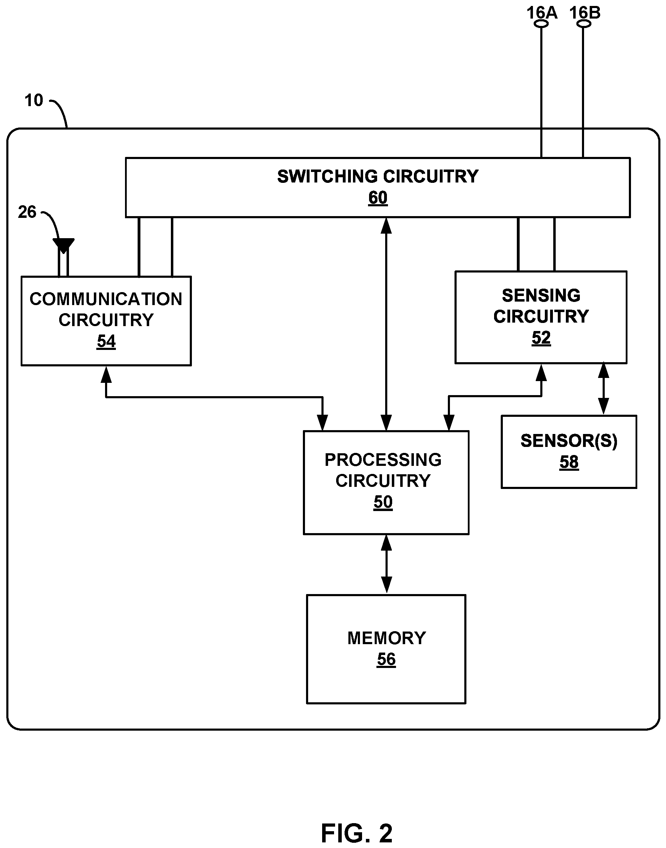

FIG. 2 is a block diagram illustrating an example of the leadless implantable medical device of FIG. 1. As shown in FIG. 2, IMD 10 includes processing circuitry 50 sensing circuitry 52, communication circuitry 54, memory 56, sensors 58, switching circuitry 60, and electrodes 16A, 16B (hereinafter "electrodes 16"), one or more of which may be disposed within a housing of IMD 10. In some examples, memory 56 includes computer-readable instructions that, when executed by processing circuitry 50, cause IMD 10 and processing circuitry 50 to perform various functions attributed to IMD 10 and processing circuitry 50 herein. Memory 56 may include any volatile, non-volatile, magnetic, optical, or electrical media, such as a random-access memory (RAM), read-only memory (ROM), non-volatile RAM (NVRAM), electrically-erasable programmable ROM (EEPROM), flash memory, or any other digital media.

Processing circuitry 50 may include fixed function circuitry and/or programmable processing circuitry. Processing circuitry 50 may include any one or more of a microprocessor, a controller, a digital signal processor (DSP), an application specific integrated circuit (ASIC), a field-programmable gate array (FPGA), or equivalent discrete or analog logic circuitry. In some examples, processing circuitry 50 may include multiple components, such as any combination of one or more microprocessors, one or more controllers, one or more DSPs, one or more ASICs, or one or more FPGAs, as well as other discrete or integrated logic circuitry. The functions attributed to processing circuitry 50 herein may be embodied as software, firmware, hardware or any combination thereof.

Sensing circuitry 52 and communication circuitry 54 may be selectively coupled to electrodes 16A, 16B via switching circuitry 60 as controlled by processing circuitry 50. Sensing circuitry 52 may monitor signals from electrodes 16A, 16B in order to monitor electrical activity of a heart of patient 4 of FIG. 1 and produce cardiac electrogram data for patient 4.

As described above, processing circuitry 50 may determine whether arrhythmia has occurred. For instance, processing circuitry 50 may perform feature delineation of the sensed cardiac electrogram data to detect bradycardia and/or pause. In some examples, processing circuitry 50 transmits, via communication circuitry 54, the cardiac electrogram data for arrhythmia episodes of patient 4 to an external device, such as external device 12 of FIG. 1. For example, IMD 10 sends digitized cardiac electrogram data to network 25 for processing by a machine learning system. In some examples, IMD 10 transmits one or more segments of the cardiac electrogram data in response to detecting, via feature delineation, an episode of arrhythmia. In another example, IMD 10 transmits one or more segments of the cardiac electrogram data in response to instructions from external device 12 (e.g., when patient 4 experiences one or more symptoms of arrhythmia and inputs a command to external device 12 instructing IMD 10 to upload the cardiac electrogram data for analysis by a monitoring center or clinician). The cardiac electrogram data may be processed by the machine learning system to detect and classify cardiac arrhythmia as described in detail below.

In some examples, IMD 10 performs feature delineation of the sensed cardiac electrogram data as described in more detail below. In some examples, the feature delineation performed by IMD 10 is of a reduced complexity so as to conserve power in IMD 10. This may enable IMD 10 to perform initial or preliminary detection of cardiac arrhythmia. As described in detail below, computing system 24 may additionally perform feature delineation of the cardiac electrogram data sensed by IMD 10, as well as apply the machine learning system to the cardiac electrogram data. Computing system 24 may possess more computational resources and less power restrictions over IMD 10, thereby allowing computing system 24 to perform a more comprehensive and detailed analysis of the cardiac electrogram data so as to more accurately detect cardiac arrhythmia. By shifting the computational burden from IMD 10 to computation system 24, the techniques of the disclosure may serve to reduce the power consumption of IMD 10 while increasing the accuracy in arrhythmia detection. Although described herein primarily in the context of examples in which computing system 24 performs the secondary analysis of episode data from IMD 10, other computing devices, such as external device 12, may additionally or alternatively perform the techniques ascribed herein to computing system.

In some examples, IMD 10 includes one or more sensors 58, such as one or more accelerometers, microphones, and/or pressure sensors. Sensing circuitry 52 may monitor signals from sensors 58 and transmit patient data obtained from sensors 58, to an external device, such as external device 12 of FIG. 1, for analysis. In some examples, sensing circuitry 52 may include one or more filters and amplifiers for filtering and amplifying signals received from one or more of electrodes 16A, 16B and/or other sensors 58. In some examples, sensing circuitry 52 and/or processing circuitry 50 may include a rectifier, filter and/or amplifier, a sense amplifier, comparator, and/or analog-to-digital converter.

Communication circuitry 54 may include any suitable hardware, firmware, software or any combination thereof for communicating with another device, such as external device 12 or another medical device or sensor, such as a pressure sensing device. Under the control of processing circuitry 50, communication circuitry 54 may receive downlink telemetry from, as well as send uplink telemetry to, external device 12 or another device with the aid of an internal or external antenna, e.g., antenna 26. In some examples, communication circuitry 54 may communicate with external device 12. In addition, processing circuitry 50 may communicate with a networked computing device via an external device (e.g., external device 12) and a computer network, such as the Medtronic CareLink^{®} Network developed by Medtronic, plc, of Dublin, Ireland.

A clinician or other user may retrieve data from IMD 10 using external device 12, or by using another local or networked computing device configured to communicate with processing circuitry 50 via communication circuitry 54. The clinician may also program parameters of IMD 10 using external device 12 or another local or networked computing device. In some examples, the clinician may select one or more parameters defining how IMD 10 senses cardiac electrogram data of patient 4.

One or more components of IMD 10 may be coupled a power source (not depicted in FIG. 2), which may include a rechargeable or non-rechargeable battery positioned within a housing of IMD 10. A non-rechargeable battery may be selected to last for several years, while a rechargeable battery may be inductively charged from an external device, e.g., on a daily or weekly basis.

In accordance with the techniques of the disclosure, processing circuitry 50 senses, with sensing circuitry 52 and via electrodes 16, cardiac electrogram data of patient 4. In some examples, the cardiac electrogram data is an ECG for patient 4. Processing circuitry 50 may determine whether arrhythmia has occurred. For example, processing circuitry 50 may determine whether bradycardia or pause has occurred based on R-R intervals (e.g., as described with respect to FIG. 1). Additionally or alternatively, processing circuitry 50 may perform feature delineation on the cardiac electrogram data to obtain one or more cardiac features present in the cardiac electrogram data. In some examples, the feature delineation includes one or more of QRS detection, refractory processing, noise processing, or delineation of the cardiac electrogram data. For example, processing circuitry 50 receives a raw signal from via sensing circuitry 52 and/or sensors 58, and extracts one or more cardiac features from the raw signal. In some examples, processing circuitry 50 identifies one or more cardiac features, such as one or more of a mean heartrate of the patient, a minimum heartrate of the patient, a maximum heartrate of the patient, a R-R interval of a heart of the patient, a variability of heartrate of the patient, one or more amplitudes of one or more features of an electrocardiogram (ECG) of the patient, or an interval between the or more features of the ECG of the patient, a T-wave alternans, QRS morphology measures, or other types of cardiac features not expressly described herein.

Processing circuitry 50 may further apply such feature delineation to determine that the one or more cardiac features are indicative of cardiac arrhythmia. Processing circuitry 50 further applies feature delineation to classify the detected episode of cardiac arrhythmia as an episode of cardiac arrhythmia of a particular type (e.g., bradycardia, tachycardia, atrial fibrillation, or ventricular fibrillation). Processing circuitry 50 transmits, via communication circuitry 54, one or more of the cardiac electrogram data, the one or more cardiac features present in the cardiac electrogram data, an indication of the detected episode of cardiac arrhythmia, or an indication of the classification of the detected episode of cardiac arrhythmia, to external device 12.

In general, a sensitive device captures arrhythmia episodes that include potential AV block episodes (i.e., both true and false-positives). For example, rate-based device triggers, e.g., for bradycardia, may not be 100% sensitive in terms of detecting all AV Block episodes. In other words, only a portion of true AV Block episodes may be detected by rate-based triggers. For instance, a device (e.g., IMD 10) with a bradycardia criteria set at 4 beats ≤ 30 beats per minute (bpm) (e.g., that 4 out of 4 consecutive heartbeats of patient 4 exhibited a heartrate of less than 30 bpm) may detect 50% of all true AV blocks; a device with a bradycardia criteria set at 4 beats ≤ 50 bpm may detect 91% of all true AV blocks; and a device with a bradycardia criteria set at 4 beats ≤ 50 bpm and pause ≥ 1.5 seconds may detect 95% of all true AV blocks.

A specific component of medical device system 2 may post-process episodes captured by a rate-based device to filter out false-positives. The typical tradeoff is between battery consumption and AV block episode sensitivity. In accordance with techniques of this disclosure, computing system 24 may select a medical device sensitivity of IMD 10 based on user input. For example, a clinician may provide user input via computing system 24 to select a sensitivity of 99% for IMD 10. Hence, if IMD 10 has a bradycardia criteria set at 4 beats ≤ 30 bpm, resulting in detection of 50% of all true AV blocks, IMD 10 may have overall AV block sensitivity of 99%*50% = 49.5%; if IMD 10 has a bradycardia criteria set at 4 beats ≤ 50 bpm, resulting in detection of 91% of all true AV blocks, IMD 10 may have overall AV block sensitivity of 99%*91% = 90.1%; if IMD 10 has a bradycardia criteria set at 4 beats ≤ 50 bpm and pause ≥ 1.5 seconds, resulting in detection of 95% of all true AV blocks, IMD 10 may have overall AV block sensitivity of 99%*95% = 94%. Medical device system 2 may present (e.g., via external device 12 or another a user interface device) the overall sensitivity (e.g., device and AV block post-processing) and specificity for detecting AV blocks for all available device programming criteria and enable physician reprogramming for the desired sensitivity.

Although described herein in the context of example IMD 10 that senses cardiac electrogram data of patient 4, the techniques for cardiac arrhythmia detection disclosed herein may be used with other types of devices. For example, the techniques may be implemented with an extra-cardiac defibrillator coupled to electrodes outside of the cardiovascular system, a transcatheter pacemaker configured for implantation within the heart, such as the Micra^{™} transcatheter pacing system commercially available from Medtronic PLC of Dublin Ireland, an insertable cardiac monitor, such as the Reveal LINQ^{™} ICM, also commercially available from Medtronic PLC, a neurostimulator, a drug delivery device, a medical device external to patient 4, a wearable device such as a wearable cardioverter defibrillator, a fitness tracker, or other wearable device, a mobile device, such as a mobile phone, a "smart" phone, a laptop, a tablet computer, a personal digital assistant (PDA), or "smart" apparel such as "smart" glasses, a "smart" patch, or a "smart" watch.

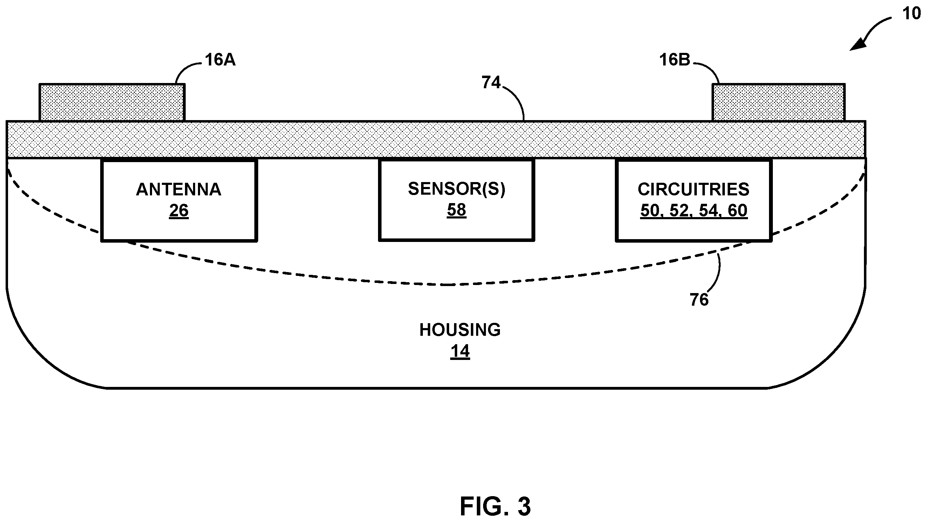

FIG. 3 is a conceptual drawing illustrating an example configuration of IMD 10. As illustrated in FIG. 3, IMD 10 may, in some examples, include a wafer-scale insulative cover 74, which may help insulate electrical signals passing between electrodes 16A, 16B on housing 14 and processing circuitry 50. In some examples, insulative cover 74 may be positioned over an open housing 14 to form the housing for the components of IMD 10. One or more components of IMD 10 (e.g., antenna 26, processing circuitry 50, sensing circuitry 52, communication circuitry 54, and/or switching circuitry 60) may be formed on a bottom side of insulative cover 74, such as by using flip-chip technology. Insulative cover 74 may be flipped onto housing 14. When flipped and placed onto housing 14, the components of IMD 10 formed on the bottom side of insulative cover 74 may be positioned in a gap 78 defined by housing 14. Housing 14 may be formed from titanium or any other suitable material (e.g., a biocompatible material), and may have a thickness of about 200 micrometers to about 500 micrometers. These materials and dimensions are examples only, and other materials and other thicknesses are possible for devices of this disclosure.

In some examples, IMD 10 collects, via sensing circuitry 52 and/or sensors 58, patient data of patient 4 including cardiac electrogram data. Sensors 58 may include one or more sensors, such as one or more accelerometers, pressure sensors, optical sensors for O2 saturation, etc. In some examples, the patient data includes one or more of an activity level of the patient, a heartrate of the patient, a posture of the patient, a cardiac electrogram of the patient, a blood pressure of the patient, accelerometer data for the patient, or other types of patient parametric data. IMD 10 uploads, via communication circuitry 54, the patient data to external device 12, which may in turn upload such data to computing system 24 over network 25. In some examples, IMD 10 uploads the patient data to computing system 24 on a daily basis. In some examples, the patient data includes one or more values that represent average measurements of patient 4 over a long-term time period (e.g., about 24 hours to about 48 hours). In this example, IMD 10 both uploads the patient data to computing system 24 and performs short-term monitoring of patient 4 (as described below). However, in other examples, the medical device that processes the patient data to detect and/or classify arrhythmia in patient 4 is different from the medical device that performs short-term monitoring of patient 4.

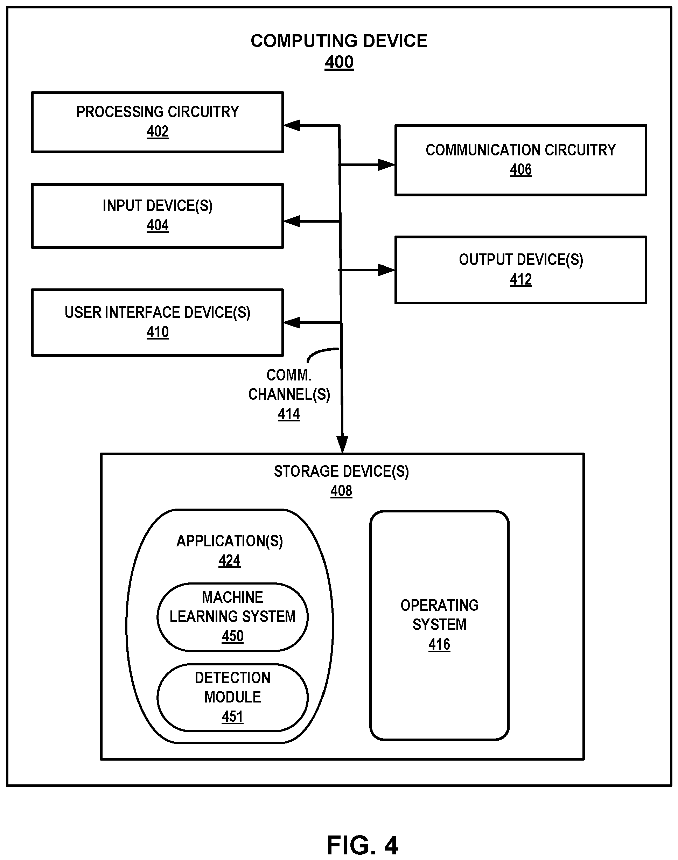

FIG. 4 is a block diagram illustrating an example computing device 400 that operates in accordance with one or more techniques of the present disclosure. In one example, computing device 400 is an example implementation of computing system 24 of FIG. 1. In another example, computing device 400 is an example implementation of external device 12.

In one example, computing device 400 includes processing circuitry 402 for executing applications 424 that include a machine learning system 450, a detection module 451, or any other applications described herein. Although shown in FIG. 4 as a stand-alone computing device 400 for purposes of example, computing device 400 may be any component or system that includes processing circuitry or other suitable computing environment for executing software instructions and, for example, need not necessarily include one or more elements shown in FIG. 4 (e.g., input devices 404, communication circuitry 406, user interface devices 410, or output devices 412; and in some examples components such as storage device(s) 408 may not be co-located or in the same chassis as other components). In some examples, computing device 400 may be a cloud computing system distributed across a plurality of devices.

As shown in the example of FIG. 4, computing device 400 includes processing circuitry 402, one or more input devices 404, communication circuitry 406, one or more storage devices 408, user interface (UI) device(s) 410, and one or more output devices 412. Computing device 400, in one example, further includes one or more application(s) 424 such as machine learning system 450, detection module 451, and operating system 416 that are executable by computing device 400. Each of components 402, 404, 406, 408, 410, and 412 are coupled (physically, communicatively, and/or operatively) for inter-component communications. In some examples, communication channels 414 may include a system bus, a network connection, an inter-process communication data structure, or any other method for communicating data. As one example, components 402, 404, 406, 408, 410, and 412 may be coupled by one or more communication channels 414.

Processing circuitry 402, in one example, is configured to implement functionality and/or process instructions for execution within computing device 400. For example, processing circuitry 402 may be capable of processing instructions stored in storage device 408. Examples of processing circuitry 402 may include, any one or more of a microprocessor, a controller, a digital signal processor (DSP), an application specific integrated circuit (ASIC), a field-programmable gate array (FPGA), or equivalent discrete or integrated logic circuitry.

One or more storage devices 408 may be configured to store information within computing device 400 during operation. Storage device 408, in some examples, is described as a computer-readable storage medium. In some examples, storage device 408 is a temporary memory, meaning that a primary purpose of storage device 408 is not long-term storage. Storage device 408, in some examples, is described as a volatile memory, meaning that storage device 408 does not maintain stored contents when the computer is turned off. Examples of volatile memories include random access memories (RAM), dynamic random access memories (DRAM), static random access memories (SRAM), and other forms of volatile memories known in the art. In some examples, storage device 408 is used to store program instructions for execution by processing circuitry 402. Storage device 408, in one example, is used by software or applications 424 running on computing device 400 to temporarily store information during program execution.

Storage devices 408, in some examples, also include one or more computer-readable storage media. Storage devices 408 may be configured to store larger amounts of information than volatile memory. Storage devices 408 may further be configured for long-term storage of information. In some examples, storage devices 408 include non-volatile storage elements. Examples of such non-volatile storage elements include magnetic hard discs, optical discs, floppy discs, flash memories, or forms of electrically programmable memories (EPROM) or electrically erasable and programmable (EEPROM) memories.

Computing device 400, in some examples, also includes communication circuitry 406. Computing device 400, in one example, utilizes communication circuitry 406 to communicate with external devices, such as IMD 10 and external device 12 of FIG. 1. Communication circuitry 406 may include a network interface card, such as an Ethernet card, an optical transceiver, a radio frequency transceiver, or any other type of device that can send and receive information. Other examples of such network interfaces may include 3G and WiFi radios.

Computing device 400, in one example, also includes one or more user interface devices 410. User interface devices 410, in some examples, are configured to receive input from a user through tactile, audio, or video feedback. Examples of user interface devices(s) 410 include a presence-sensitive display, a mouse, a keyboard, a voice responsive system, video camera, microphone or any other type of device for detecting a command from a user. In some examples, a presence-sensitive display includes a touch-sensitive screen.

One or more output devices 412 may also be included in computing device 400. Output device 412, in some examples, is configured to provide output to a user using tactile, audio, or video stimuli. Output device 412, in one example, includes a presence-sensitive display, a sound card, a video graphics adapter card, or any other type of device for converting a signal into an appropriate form understandable to humans or machines. Additional examples of output device 412 include a speaker, a cathode ray tube (CRT) monitor, a liquid crystal display (LCD), or any other type of device that can generate intelligible output to a user.

Computing device 400 may include operating system 416. Operating system 416, in some examples, controls the operation of components of computing device 400. For example, operating system 416, in one example, facilitates the communication of one or more applications 424 and long-term prediction module 450 with processing circuitry 402, communication circuitry 406, storage device 408, input device 404, user interface devices 410, and output device 412.

Application 422 may also include program instructions and/or data that are executable by computing device 400. Example application(s) 422 executable by computing device 400 may include machine learning system 450 and detection module 451. Other additional applications not shown may alternatively or additionally be included to provide other functionality described herein and are not depicted for the sake of simplicity.

In accordance with the techniques of the disclosure, computing device 400 may execute one or more applications 422 to determine whether AV block has occurred. For example, computing device 400 may apply a machine learning model of machine learning system 450 to patient data sensed by IMD 10 to detect and classify an episode of arrhythmia occurring in patient 10. In another example, computing device 400 may execute detection module 451 to determine whether AV block has occurred based on P-R intervals (e.g., as described in greater detail with respect to FIG. 1).

In some examples, the machine learning model implemented by machine learning system 450 is trained with training data that comprises cardiac electrogram data for a plurality of patients labeled with descriptive metadata. For example, during a training phase, machine learning system 450 processes a plurality of ECG waveforms. Typically, the plurality of ECG waveforms are from a plurality of different patients. Each ECG waveform is labeled with one or more episodes of arrhythmia of one or more types. For example, a training ECG waveform may include a plurality of segments, each segment labeled with a descriptor that specifies an absence of arrhythmia or a presence of an arrhythmia of a particular classification (e.g., bradycardia, tachycardia, atrial fibrillation, ventricular fibrillation, or AV Block). In some examples, a clinician labels the presence of arrhythmia in each ECG waveform by hand. In some examples, the presence of arrhythmia in each ECG waveform is labeled according to classification by feature delineation. Machine learning system 450 may operate to convert the training data into vectors and tensors (e.g., multidimensional arrays) upon which machine learning system 450 may apply mathematical operations, such as linear algebraic, nonlinear, or alternative computation operations. Machine learning system 450 uses the training data to teach the machine learning model to weigh different features depicted in the cardiac electrogram data. In some examples, machine learning system 450 uses the cardiac electrogram data to teach the machine learning model to apply different coefficients that represent one or more features in a cardiac electrogram as having more or less importance with respect to an occurrence of a cardiac arrhythmia of a particular classification. By processing numerous such ECG waveforms labeled with episodes of arrhythmia, machine learning system 450 may build and train a machine learning model to receive cardiac electrogram data from a patient, such as patient 4 of FIG. 1, that machine learning system 450 has not previously analyzed, and process such cardiac electrogram data to detect the presence or absence of arrhythmia of different classifications in the patient with a high degree of accuracy. Typically, the greater the amount of cardiac electrogram data on which machine learning system 450 is trained, the higher the accuracy of the machine learning model in detecting or classifying cardiac arrhythmia in new cardiac electrogram data.

After machine learning system 450 has trained the machine learning model, machine learning system 450 may receive patient data, such as cardiac electrogram data, for a particular patient, such as patient 4. Machine learning system 450 applies the trained machine learning model to the patient data to detect an occurrence of an episode of cardiac arrhythmia in patient 4. Further, machine learning system 450 applies the trained machine learning model to the patient data to classify the episode of cardiac arrhythmia in patient as indicative of a particular type of arrhythmia. In some examples, machine learning system 450 may output a preliminary determination that the episode of cardiac arrhythmia is indicative of a particular type of arrhythmia, as well as an estimate of certainty in the determination. In response to determining that the estimate of certainty in the determination is greater than a predetermined threshold (e.g., 50%, 75%, 90%, 95%, 99%), computing device 400 may classify that the episode of cardiac arrhythmia as the particular type of arrhythmia.

In some examples, machine learning system may process one or more cardiac features of cardiac electrogram data instead of the raw cardiac electrogram data itself. The one or more cardiac features may be obtained via feature delineation. IMD 10 may perform the feature delineation, as described above. Additionally or alternatively, another component of medical system 2, such as processing circuitry 102, may perform feature delineation in a manner similar to that described above. The cardiac features may include, e.g., one or more of a mean heartrate of the patient, a minimum heartrate of the patient, a maximum heartrate of the patient, a PR interval of a heart of the patient, a variability of heartrate of the patient, one or more amplitudes of one or more features of an electrocardiogram (ECG) of the patient, or an interval between the or more features of the ECG of the patient, a T-wave alternans, QRS morphology measures, or other types of cardiac features not expressly described herein. In such example implementations, machine learning system may train the machine learning model via a plurality of training cardiac features labeled with episodes of arrhythmia, instead of the plurality of ECG waveforms labeled with episodes of arrhythmia as described above.

In some examples, machine learning system 450 may apply the machine learning model to other types of data to determine that an episode of arrhythmia has occurred in patient 4. For example, machine learning system 450 may apply the machine learning model to one or more characteristics of cardiac electrogram data that are correlated to arrhythmia in the patient, an activity level of IMD 10, an input impedance of IMD 10, or a battery level of IMD 10.

In further examples, processing circuitry 402 may generate, from the cardiac electrogram data, an intermediate representation of the cardiac electrogram data. For example, processing circuitry 402 may apply one or more signal processing, signal decomposition, wavelet decomposition, filtering, or noise reduction operations to the cardiac electrogram data to generate the intermediate representation of the cardiac electrogram data. In this example, machine learning system 450 processes such an intermediate representation of the cardiac electrogram data to detect and classify an episode of arrhythmia in patient 4. Furthermore, machine learning system may train the machine learning model via a plurality of training intermediate representations labeled with episodes of arrhythmia, instead of the plurality of raw ECG waveforms labeled with episodes of arrhythmia as described above. The use of such intermediate representations of the cardiac electrogram data may allow for the training and development of a lighter-weight, less computationally complex machine learning model by machine learning system 450. Further, the use of such intermediate representations of the cardiac electrogram data may require less iterations and fewer training data to build an accurate machine learning model, as opposed to the use of raw cardiac electrogram data to train the machine learning model.

In some examples, computing device 400 may use machine learning system 450 to detect other types of arrhythmias beyond the ones detected in the feature delineation screening analysis. For example, arrhythmia detection by performing feature delineation implemented by low-power devices such as IMD 10 may not be designed to detect less-frequently occurring arrhythmias, such as AV Blocks. The machine learning system may train a machine learning model on large datasets where such arrhythmias are available, thereby providing finer granularity and higher accuracy over feature delineation performed by, e.g., IMD 10 alone. Therefore, the use of machine learning system 450 may expand the arrhythmia diagnosis capability of system 2 by allowing IMD 10 to implement screening using feature delineation followed by the use of machine learning system 450 that implements a machine learning model that can provide a wider range of arrhythmia detection. After detecting a type of arrhythmia that was not detected by feature delineation, computing system 24 may nevertheless use feature delineation, such as QRS detection, to assist in characterizing and reporting the other types of arrhythmias detected by the machine learning model of machine learning system 450.

In some examples, computing system 24 may tailor machine learning system 450 to the specific use case. For example, machine learning system 450 may implement a machine learning model specific to detecting AV Blocks and bradycardia where patient 4 is a post-TAVR patient. As another example, machine learning system 450 may implement a machine learning model specific to detecting PVCs such that PVC burden may be used to risk-stratify patients who might be indicated for ICDs.

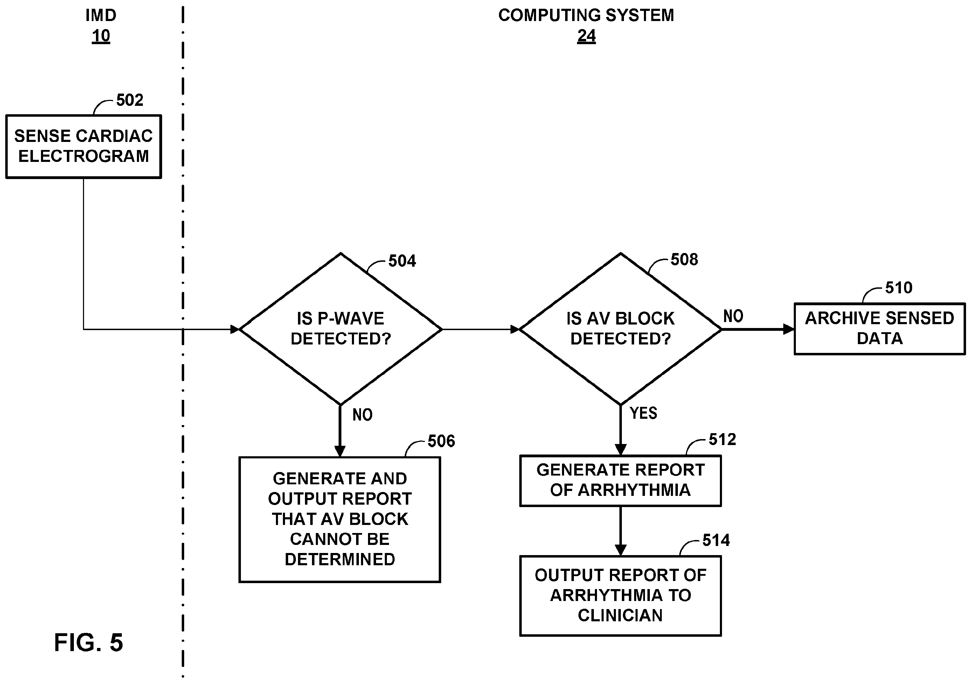

FIG. 5 is a flowchart illustrating an example operation in accordance with the techniques of the disclosure. For convenience, FIG. 5 is described with respect to FIG. 1. In some examples, the operation of FIG. 5 is an operation for detecting and classifying cardiac arrhythmia in patient 4.

As depicted in FIG. 5, IMD 10 senses cardiac electrogram data of patient 4 (502). The cardiac electrogram data can be, e.g., an episodic ECG of patient 4 or a full-disclosure ECG of patient 4. Further, the cardiac electrogram data of patient 4 may be from a single-channel or multi-channel system. For simplicity, in the example of FIG. 5, the cardiac electrogram data of patient 4 is described as single-channel episodic ECG data.

Processing circuitry 102 of computing system 24 may receive the cardiac electrogram data and identify a plurality of heartbeats within the cardiac electrogram data for the episode. Processing circuitry 102 may analyze each heartbeat of the plurality of heartbeats. In some examples, processing circuitry 102 may determine R-R intervals for the plurality of heartbeats.

Processing circuitry 102 may determine whether a P-wave is detectable in the cardiac electrogram data (504). Responsive to determining that the P-wave is detectable ("YES" block of 504), processing circuitry 102 may determine a P-wave location in the cardiac electrogram data and a P-R interval. Responsive to determining that one or more P-waves are not detectable, processing circuitry 102 may generate and output a report comprising an indication that whether AV block has occurred during the episode is indeterminate (506).

Responsive to determining that the P-wave is detectable ("YES" block of 504), processing circuitry 102 may determine whether AV block has occurred (508). For instance, processing circuitry 102 may determine whether AV block has occurred based on the determined P-R intervals. For example, if processing circuitry 102 determines that one of the P-R intervals exceeds 200 ms (or another appropriate threshold value) but the 1:1 P-wave-to-QRS-complex ratio is maintained, processing circuitry 102 may determine that a first-degree AV block has occurred during the episode recorded by IMD 10. In another example, if processing circuitry 102 determines that the P-R intervals indicate that a 1:1 P-wave-to-QRS-complex ratio is not maintained, processing circuitry 102 may determine that a second-degree AV block has occurred. In the same example, if the P-R intervals become progressively longer, processing circuitry 102 may determine that a type 1 second-degree AV block (i.e., Mobitz I) has occurred. Otherwise, processing circuitry 102 may determine that a type 2 second-degree AV block (i.e., Mobitz II) has occurred. In yet another example, if processing circuitry 102 determines, based on the P-R intervals and R-R intervals, that the P-waves occur at one rate and the QRS complexes at another, processing circuitry 102 may determine that a third-degree AV block has occurred.

In some examples, processing circuitry 102 may exclude specific heartbeats when determining whether AV block has occurred based on P-R intervals. For example, processing circuitry 102 may exclude P-R intervals for heartbeats that adjacently follow a premature ventricular contraction heartbeat such that processing circuitry 102 cannot determine whether AV block has occurred based on P-R intervals for heartbeats that adjacently follow a premature ventricular contraction heartbeat. In another example, processing circuitry 102 may determine whether the R-R interval of each heartbeat satisfies an R-R interval threshold. Processing circuitry 102 may exclude P-R intervals for heartbeats for which the R-R interval threshold is not satisfied.

Responsive to determining that AV block has not occurred ("NO" block of 508), processing circuitry may archive the cardiac electrogram data (510) for subsequent review by a clinician. Responsive to determining that AV block has occurred ("YES" block of 508), processing circuitry 102 may generate a report (512). The report may include an indication that AV block has occurred in patient 4. The report may further include at least one of the R-R intervals or the P-R intervals that coincide with the AV block that has occurred during the episode. In this way, the techniques of this disclosure may provide a clinician with information that explains the determination of AV block, thereby potentially increasing confidence in the accuracy of the diagnosis. Processing circuitry 102 may output the report to a clinician or monitoring center (514).

In some examples, processing circuitry 102 may select a medical device sensitivity of IMD 10. For example, a clinician may provide user input via computing system 24 to select a sensitivity of 99% for IMD 10. Hence, if IMD 10 has a brady criteria set at 4 beats ≤ 30 bpm, resulting in detection of 50% of all true AV blocks, IMD 10 may have overall AV block sensitivity of 99%*50% = 49.5%; if IMD 10 has a brady criteria set at 4 beats ≤ 50 bpm, resulting in detection of 91% of all true AV blocks, IMD 10 may have overall AV block sensitivity of 99%*91% = 90.1%; if IMD 10 has a brady criteria set at 4 beats ≤ 50 bpm and pause ≥ 1.5 seconds, resulting in detection of 95% of all true AV blocks, IMD 10 may have overall AV block sensitivity of 99%*95% = 94%. Medical device system 2 may present (e.g., via a user interface device) the overall sensitivity (e.g., device and AV block post-processing) and specificity for detecting AV blocks for all available device programming criteria and enable physician reprogramming for the desired sensitivity.

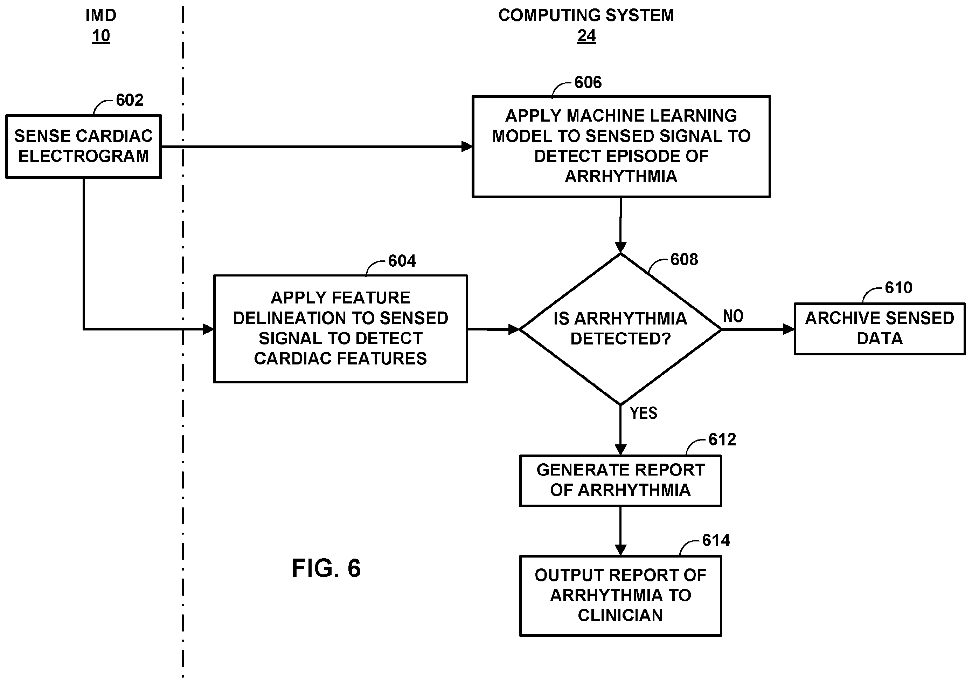

FIG. 6 is a flowchart illustrating an example operation in accordance with the techniques of the disclosure. For convenience, FIG. 6 is described with respect to FIG. 1. In some examples, the operation of FIG. 6 is an operation for detecting and classifying cardiac arrhythmia in patient 4. In the operation of FIG. 6 processing circuitry 102 of computing system 24 combines ability of a machine learning model of a machine learning system to learn features and perform classification directly from an input with the interpretability provided by the feature delineation and ECG-processing. In the example operation of FIG. 6, system 2 implements a machine learning model of a machine learning system in parallel with feature delineation to perform arrhythmia detection and characterization.

As depicted in FIG. 6, IMD 10 senses cardiac electrogram data of patient 4 (602). The cardiac electrogram data can be, e.g., an episodic ECG of patient 4 or a full-disclosure ECG of patient 4. Further, the cardiac electrogram data of patient 4 may be from a single-channel or multi-channel system. For simplicity, in the example of FIG. 6, the cardiac electrogram data of patient 4 is described as single-channel episodic ECG data.

Processing circuitry 102 of computing system 24 may receive the cardiac electrogram data and identify a plurality of heartbeats within the cardiac electrogram data for the episode. Processing circuitry 102 may analyze each heartbeat of the plurality of heartbeats. In some examples, processing circuitry 102 may determine R-R intervals.

In some examples, processing circuitry 102 may determine whether a P-wave is detectable in the cardiac electrogram data. Responsive to determining that the P-wave is detectable, processing circuitry 102 may determine a P-wave location in the cardiac electrogram data and a P-R interval. In some examples, responsive to determining that one or more P-waves are not detectable, processing circuitry 102 may generate and output a report comprising an indication that whether AV block has occurred during the episode is indeterminate.

Processing circuitry 102 may execute a machine learning system to apply a machine learning model to the sensed cardiac electrogram to detect an episode of arrhythmia, such as AV block, in patient 4 (606). In some examples, the machine learning model is trained with a plurality of ECG episodes annotated by a clinician or a monitoring center for arrhythmias of several different types. In one example, the machine learning system applies the machine learning model to take one or several subsegments of a normalized input ECG signal and generates arrhythmia labels and a likelihood of an occurrence of the arrhythmia. In some examples, the machine learning model may be accurate in mapping an input ECG to an output arrhythmia label, but may not provide additional arrhythmia characteristics or identify the specific cardiac features, such as a mean heartrate, a maximum heartrate, P-R interval characteristics, etc., used to make the determination that an episode of arrhythmia has occurred in patient 4. Furthermore, one may be unable to obtain physician-provided notifications or reportable criteria (e.g., a bradycardia criteria) from the output or intermediate states of the machine learning model such that a clinician would be able to make use of the determination that an episode of arrhythmia has occurred in patient 4 for use in providing subsequent therapy to patient 4.

To address this, computing system 24 may also apply feature delineation to the cardiac electrogram data to detect one or more cardiac features (604). In some examples, computing system 24 further applies feature delineation to the cardiac electrogram data to detect one or more episodes of arrhythmia. For example, computing system 24 may apply QRS detection delineation and noise flagging (e.g., is the beat noisy or not) to the cardiac electrogram data to provide arrhythmia characteristics and/or cardiac features for detected episodes of arrhythmia (e.g., an average heartrate during an episode of atrial fibrillation, a duration of a pause). Further, computing system 24 may apply feature delineation to guide notification and reporting criteria for system 2. In the example of FIG. 6, computing system 24 performs feature delineation of the cardiac electrogram data. However, in other examples of the techniques of the disclosure, other devices, such as IMD 10, external device 12, or another external medical device, may perform feature delineation of the cardiac electrogram data.

With respect to the example of FIG. 6, computing system 24 applies both the machine learning system and feature delineation to determine whether an episode of cardiac arrhythmia is detected in patient 4 (608). If neither the machine learning system nor feature delineation detect an episode of cardiac arrhythmia (e.g., "NO" block of 608), then computing system 24 may archive the cardiac electrogram data for subsequent review by a clinician (610).

If at least one of the machine learning system or the feature delineation operation of (604) detects an episode of cardiac arrhythmia (e.g., "YES" block of 608), then computing system may generate a report of the arrhythmia (612) and output the report to a clinician or monitoring center (614). As described above, the report may include an indication that cardiac arrhythmia, such as AV block, has occurred in patient 4. The report may further include at least one of the R-R intervals or the P-R intervals that coincide with the cardiac arrhythmia that has occurred during the episode. In this way, the techniques of this disclosure may provide a clinician with information that explains the determination of AV block, thereby potentially increasing confidence in the accuracy of the diagnosis.

In some examples, the report includes an indication that the episode of arrhythmia has occurred in the patient and one or more of the cardiac features that coincide with the episode of arrhythmia. In some examples, the report further includes a classification of the episode of arrhythmia as a particular type of arrhythmia (e.g., AV block). In some examples, the report includes a subsection of the cardiac electrogram data obtained from patient 4 that coincides with the episode of arrhythmia. For example, computing system 24 may identify a subsection of the cardiac electrogram data of patient 4, wherein the subsection comprises cardiac electrogram data for a first time period prior to the episode of arrhythmia (e.g., typically less than 10 minutes prior to the onset of the episode of arrhythmia), a second time period during the occurrence of the episode of arrhythmia, and a third time period after the episode of arrhythmia (e.g., typically less than 10 minutes after the cessation of the episode of arrhythmia). As an example, a subsection of the cardiac electrogram data of patient 4 may be about 6 seconds in length and includes representative segments before, during, and after an episode of arrhythmia (if present in the cardiac electrogram data or waveform that is analyzed). In some examples, the episode duration differs by device type, and may further depend on a use case for the medical device, one or more settings of the medical device, or a particular type of arrhythmia sensed. For example, some types of arrhythmia self-terminate quickly, (resulting in a short duration episode), while other types of arrhythmia are sustained and of a length such that the recorded duration of the episode may depend on a designated memory space on the medical device. As an example, for atrial fibrillation (AF), the subsection of the cardiac electrogram data of patient 4 may include cardiac electrogram data during an onset time period, a segment of maximum AF likelihood, a segment of fastest AF rate, and an AF offset. Typically, a length of time of the cardiac electrogram data of the patient is greater than the first, second, and third time periods. Further, computing system 24 identifies one or more of the cardiac features that coincide with the first, second, and third time periods. Computing system 24 includes, in the report, the subsection of the cardiac electrogram data and the one or more of the cardiac features that coincide with the first, second, and third time periods.

In some examples, computing system 24 receives, from a clinician, one or more adjustments to an operation to the feature-based delineation of the cardiac electrogram data that are based on the report. Computing device 24 subsequently may perform feature-based delineation of the cardiac electrogram data of patient 4 in accordance with the one or more adjustments.

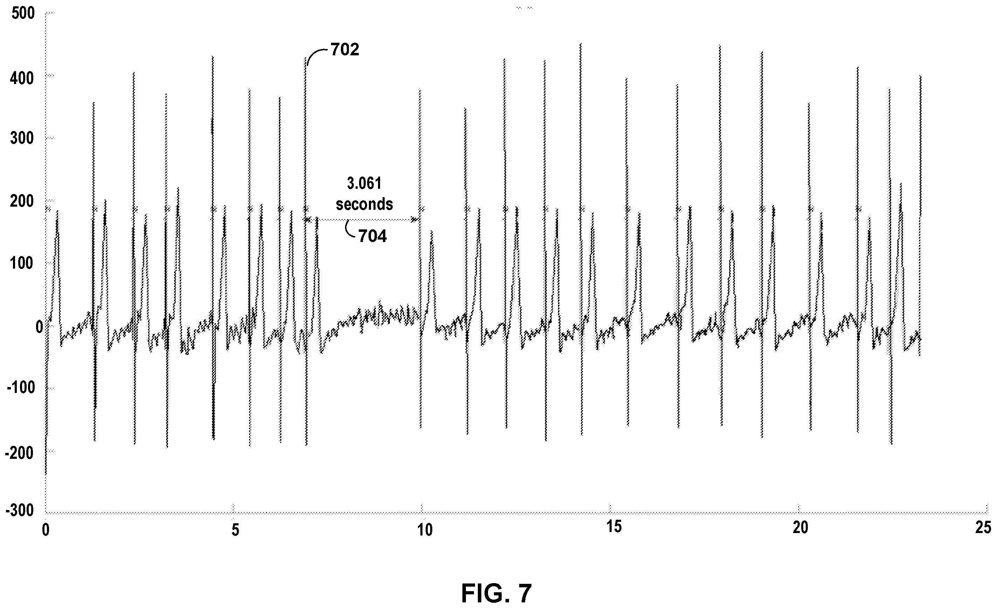

FIG. 7 is a chart illustrating example electrocardiogram 702 obtained from patient 4 of FIG. 1. Electrocardiogram 702 may be sensed, for example, by sensing circuitry 52 of IMD 10. Processing circuitry 102 of computing system 24 (and/or any other processing circuitry of system 2) may execute a machine learning system to apply a machine learning model to electrocardiogram 702 to determine that electrocardiogram 702 includes pause 704. In some examples, computing system 24 of FIG. 1 or IMD 10 of FIG. 1 (e.g., as part of IMD 10 initially detecting an arrhythmia) may perform feature delineation on electrocardiogram 702 to determine a length of pause 704. With respect to the example of FIG. 7, computing system 24 or IMD 10 determines, via feature delineation of electrocardiogram 702, that pause 704 has a length of 3.061 seconds. In one example, IMD 10 performs QRS detection from an on-device marker channel. The QRS flagging may be based on conventional QRS identification techniques. IMD 10 may use QRS markers to determine that the pause duration is 3.061 seconds.

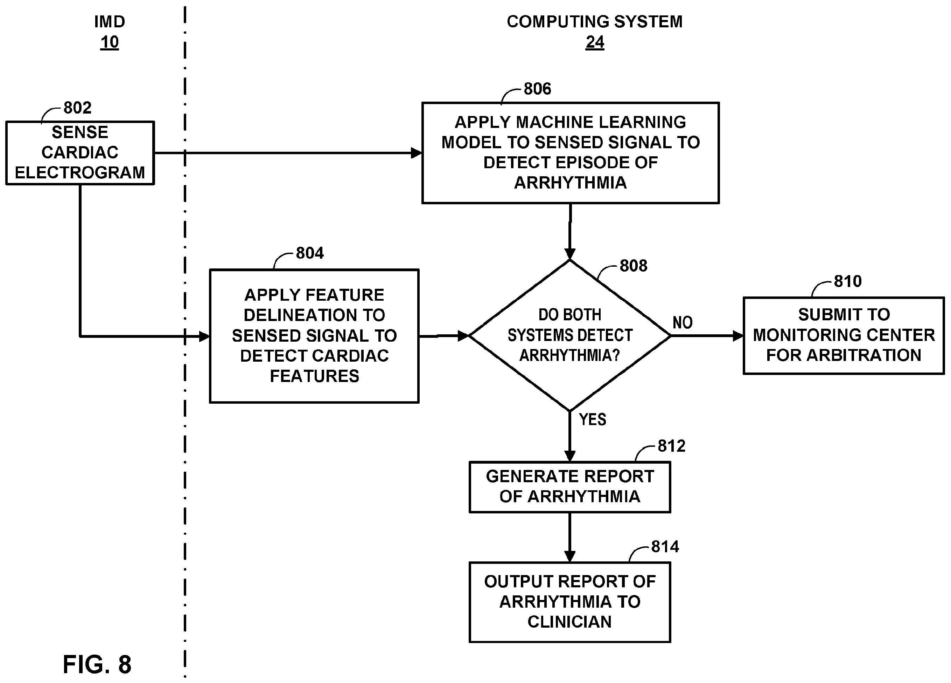

FIG. 8 is a flowchart illustrating an example operation in accordance with the techniques of the disclosure. For convenience, FIG. 8 is described with respect to FIG. 1. The operation of FIG. 8 is an operation for detecting and classifying cardiac arrhythmia in patient 4. Specifically, the operation of FIG. 8 depicts an implementation where computing system 24 uses machine learning arrhythmia detection of the machine learning system and feature delineation in parallel to perform cardiac arrhythmia detection, verification, and reporting.

As depicted in FIG. 8, IMD 10 senses cardiac electrogram data of patient 4 (802). Computing system 24 applies feature delineation to the cardiac electrogram data to detect one or more cardiac features (804). In the example of FIG. 8, computing system 24 performs feature delineation of the cardiac electrogram data. However, in other examples of the techniques of the disclosure, other devices, such as IMD 10, external device 12, or another external medical device, may perform feature delineation of the cardiac electrogram data. The machine learning system of computing system 24 applies a machine learning model to the sensed cardiac electrogram to detect an episode of arrhythmia in patient 4 (806). The operation of steps 802, 804, and 806 may occur in a substantially similar fashion to steps 502, 504, and 506 of FIG. 5, respectively.

Computing system 24 determines whether both the machine learning system and the feature delineation operation of (804) detect an episode of cardiac arrhythmia (808). For example, computing system 24 may determine a level of confidence that the determination of arrhythmia by the machine learning system matches the determination of arrhythmia by the feature delineation operation of 804 (808). For example, if computing system 24 determines that both the machine learning system and the feature delineation operation of (804) detect an episode of cardiac arrhythmia (e.g., "YES" block of 808), then computing system 24 may generate a report of the arrhythmia (812) and outputs the report to a clinician or monitoring center (814). For example, computing system 24 populates a report with the detected arrhythmias along with the arrhythmia characteristics and outputs the report to the clinician. The operation of steps 812 and 814 may occur in a substantially similar fashion to steps 512 and 514 of FIG. 5, respectively.

As another example, if computing system 24 determines that the machine learning system and the feature delineation operation of (804) disagree as to whether an episode of cardiac arrhythmia is detected (e.g., "NO" block of 808), then computing system 24 submits the cardiac electrogram data to a monitoring center for arbitration, e.g., by a human reviewer (810). In other words, computing system 24 presents the cardiac electrogram data for human overview where there is a discrepancy between the two detection methods. Such a workflow may allow for the reduction in human review burden to only those arrhythmias that computing system 24 is unable to evaluate with a high degree of confidence. For example, if the arrhythmias detected via feature delineation are similar to arrhythmias independently detected by the machine learning model, then computing system 24 may determine that the arrhythmias detected via feature delineation are independently verified without requiring expert human review. Thus, the techniques of the disclosure may reduce the amount of review required by clinicians and/or experts, thereby reducing the administrative overhead and cost of cardiac monitoring of patient 4.

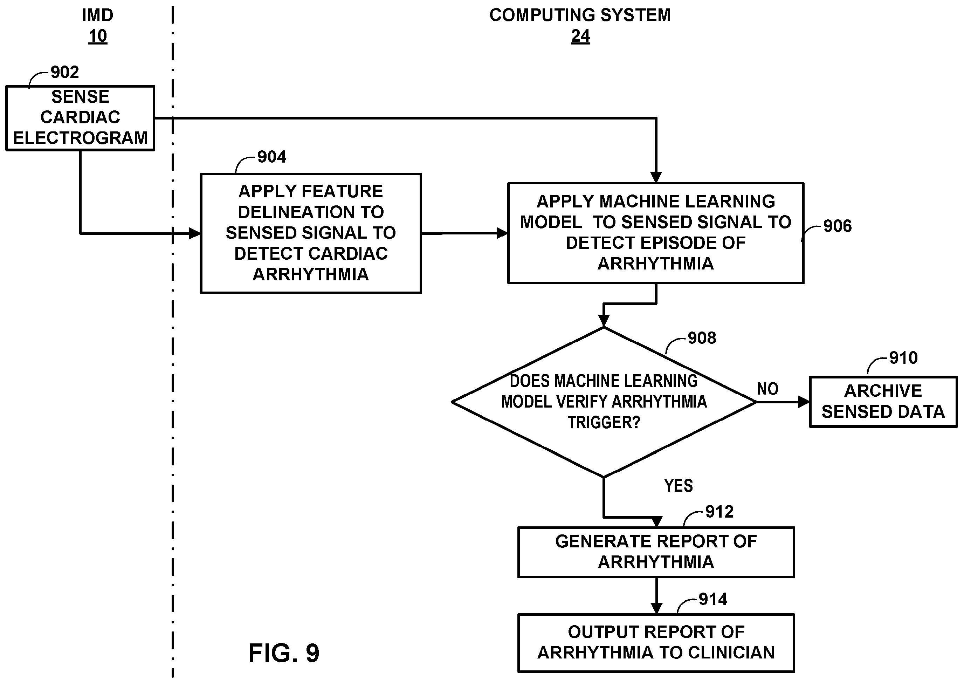

FIG. 9 is a flowchart illustrating an example operation in accordance with the techniques of the disclosure. For convenience, FIG. 9 is described with respect to FIG. 1. The operation of FIG. 9 is an operation for detecting and classifying cardiac arrhythmia in patient 4. Specifically, the operation of FIG. 9 depicts an implementation where computing system 24 uses feature delineation in series with machine learning arrhythmia detection of a machine learning system to perform cardiac arrhythmia detection, verification, and reporting.

As depicted in FIG. 9, IMD 10 senses cardiac electrogram data of patient 4 (902). The operation of step 902 may occur in a substantially similar fashion to step 502 of FIG. 5. Computing system 24 applies feature delineation to the cardiac electrogram data to detect a set of cardiac arrhythmias and one or more cardiac features (904). In some examples, computing system 24 applies feature delineation to detect arrhythmia such as bradycardia, tachycardia, pause, or atrial fibrillation based on rate and variability features in the cardiac electrogram data. In the example of FIG. 9, computing system 24 performs feature delineation as a screening step before delineating all arrhythmias (e.g., computing system 24 may use feature delineation to consider only tachyarrhythmia with heartrates greater than or equal to 120 BPM, bradyarrhythmia with heartrates less than or equal to 40 BPM, or arrhythmias with high RR variability). In other examples, such feature delineation may be implemented on low-power devices such as IMD 10 or other types of devices, such as external device 12 or another external medical device.

Upon detecting via feature delineation that an episode of cardiac arrhythmia has occurred in patient 4, a machine learning system executed by processing circuitry 102 of computing system 24 applies a machine learning model to the sensed cardiac electrogram to verify that the episode of arrhythmia has occurred (906). In some examples, the machine learning system applies the machine learning model to many different types of patient data, such as the cardiac electrogram data for patient 4, the trigger reason that caused feature delineation to detect an arrhythmia, one or more types of arrhythmias detected by feature delineation, or device characteristics of IMD 10 such as activity level, input impedance, battery level, etc.

In the example of FIG. 9, computing system 24 determines whether the machine learning system verifies the arrhythmia trigger of the feature delineation of step 904 (908). In other words, in response to determining that the feature delineation of step 904 has detected an episode of arrhythmia in patient 4, computing system 24 determines whether the machine learning system likewise detects an episode of arrhythmia in patient 4. The use of the machine learning system allows computing system 24 to verify whether the detection reason of the feature delineation of step 904 was appropriate (e.g., a bradycardia trigger of the feature delineation was truly indicative that an episode of bradycardia in patient 4 has occurred). The use of the machine learning system as a verification tool may assist computing system 24 in providing feedback to physicians for re-programming diagnostic devices for patient 4, such as IMD 10. Further, the use of the machine learning system as a verification tool may assist computing system 24 in automating the reporting of physiological parameters (e.g., report the device-detected AF burden as-is if all AF triggered episodes are appropriate, else, only consider the burden for appropriately-triggered episodes).

For example, if computing system 24 determines that the machine learning system verifies the detection of the episode of cardiac arrhythmia by the feature delineation operation of 904 (e.g., "YES" block of 908), then computing system 24 may generate a report of the arrhythmia (912) and outputs the report to a clinician or monitoring center (914). As another example, if computing system 24 determines that the machine learning system and the feature delineation operation of 904 disagree as to whether an episode of cardiac arrhythmia is detected (e.g., "NO" block of 908), then computing system 24 submits the cardiac electrogram data to a monitoring center for arbitration (910). The operation of steps 910, 912, and 914 may occur in a substantially similar fashion to steps 510, 512, and 514 of FIG. 5, respectively.

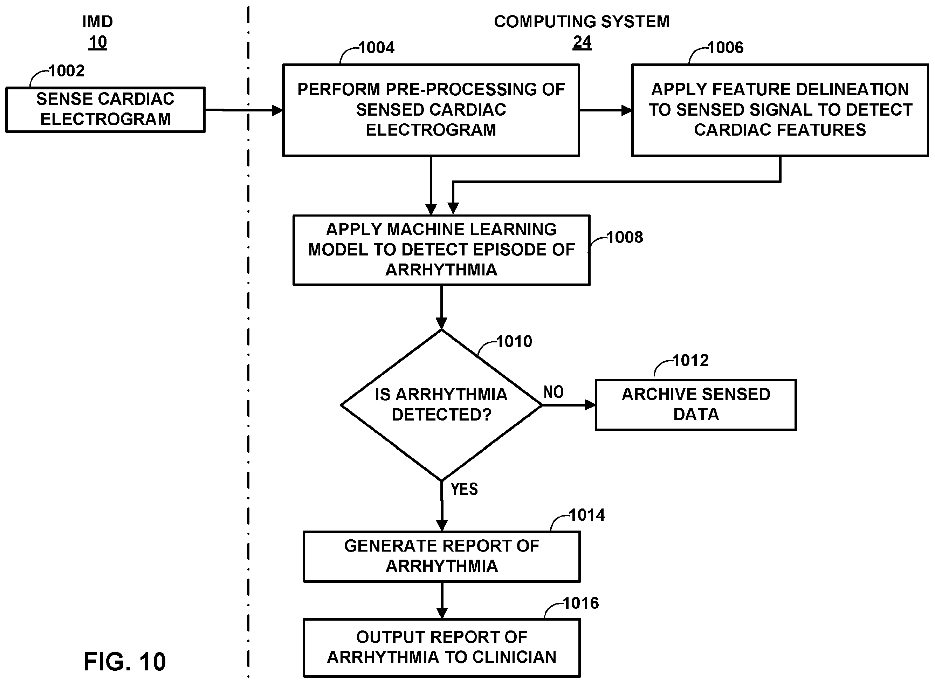

FIG. 10 is a flowchart illustrating an example operation in accordance with the techniques of the disclosure. For convenience, FIG. 10 is described with respect to FIG. 1. The operation of FIG. 10 is an operation for detecting and classifying cardiac arrhythmia in patient 4. Specifically, the operation of FIG. 10 depicts an implementation where computing system 24 preprocesses the cardiac electrogram data to generate an intermediate representation of the cardiac electrogram data, and applies a machine learning system to the intermediate representation of the cardiac electrogram data to perform cardiac arrhythmia detection, verification, and reporting.

In the example of FIG. 10, IMD 10 senses cardiac electrogram data of patient 4 (1002). The operation of step 1002 may occur in a substantially similar fashion to step 502 of FIG. 5. Computing system 24 performs pre-processing of the sensed cardiac electrogram data to generate an intermediate representation of the cardiac electrogram data (1004). For example, computing system 24 performs QRS detection to detect a plurality of QRS windows within the sensed cardiac electrogram data. In one example, the window around the detected QRS includes data for 160 milliseconds prior to the detected QRS and data for 160 milliseconds after the detected QRS. In another example, the window around the detected QRS includes a data segment from a T-offset of a previous QRS to a T-offset of the current QRS. In some examples, computing system 24 may apply signal processing methods such as bandpass filtering or stationary wavelet decomposition that are used for QRS detection, flagging and delineation to the sensed cardiac electrogram data. For example, computing system 24 generates a wavelet decomposition of the cardiac electrogram of patient 4 for the window around the detected QRS.

Computing system 24 applies feature delineation to the intermediate representation of the cardiac electrogram data to detect one or more cardiac features (1006). For example, computing system 24 applies feature delineation to the intermediate representation to detect and delineate a QRS segment (e.g., P-R intervals) of patient 4 from the window around the detected QRS, as well as a noise flag. In the example of FIG. 10, computing system 24 performs feature delineation of the cardiac electrogram data. However, in other examples of the techniques of the disclosure, other devices, such as IMD 10, external device 12, or another external medical device, may perform feature delineation of the cardiac electrogram data.

The machine learning system executed by processing circuitry 102 of computing system 24 may apply a machine learning model to the intermediate representation of the sensed cardiac electrogram to detect an episode of arrhythmia in patient 4 (1008). For example, the machine learning model may receive, as an input, a plurality of cardiac electrogram segments, each segment including a window around a detected QRS, a QRS delineation for the segment, and a noise flag for the segment. The machine learning system applies the machine learning model to the received segments to detect an episode of arrhythmia in patient 4.

In some examples, the machine learning model is tuned to capture segments of interest of each arrhythmia. For example, the machine learning model may process the sensed cardiac electrogram to capture an onset, an offset, a highest heartrate, and a lowest heartrate from the segment including the window around the detected QRS. In some examples, computing system 24 uses features derived from feature delineation such as QRS detection, such as the heartrate values of the cardiac electrogram segment, to characterize or contextualize a detection of arrhythmia by the machine learning model.

The use of signal decomposition to create the intermediate representation of the cardiac electrogram may allow for the use existing knowledge about the frequency bands of interest for arrhythmia detection. Further, the signal decomposition may limit the computational complexity of the machine learning model of the machine learning system such that the machine learning model may learn features for classification from only the cardiac electrogram subsegments corresponding to the detected QRS. Thus, such techniques may reduce the complexity of the machine learning model, allowing for a reduction in the size of the training set needed to generate the machine learning model as well as increasing the accuracy in the machine learning model.

In contrast to the operation of FIG. 5, computing system 24 may use the same signal pre-processing for both feature delineation detection of cardiac arrhythmia and/or cardiac features of step 1006 and the machine learning model detection of cardiac arrhythmia. Furthermore, computing system 24 may use the QRS noise-flag and feature delineation as inputs for the machine learning model of the machine learning system. The input cardiac electrogram complexes may be of the same duration (e.g., 320 milliseconds) or of different durations (e.g., the segment from the previous T-offset to the current T-offset).

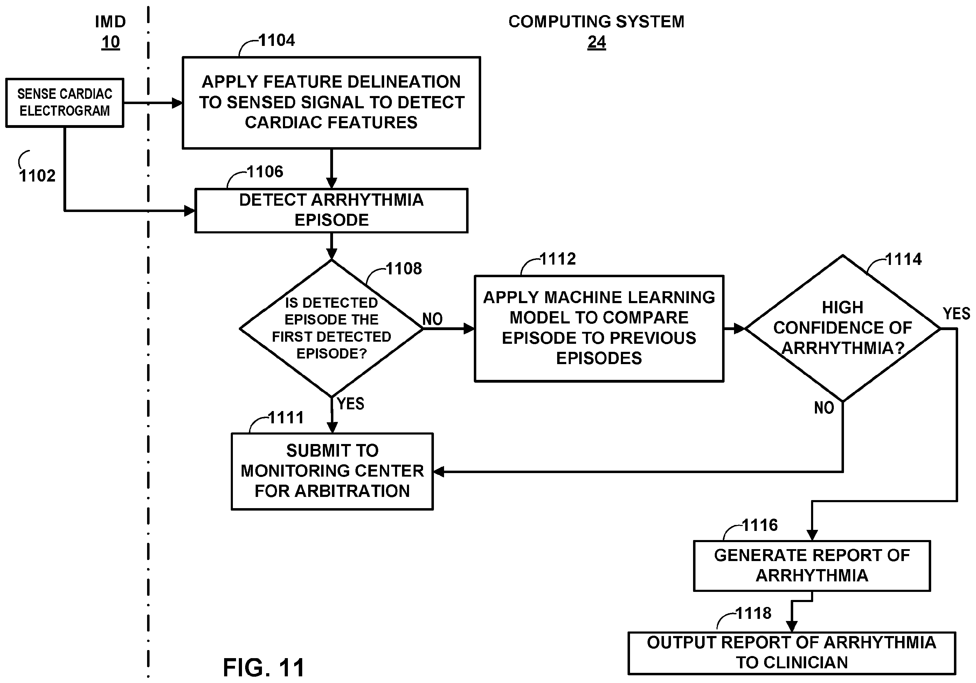

FIG. 11 is a flowchart illustrating an example operation in accordance with the techniques of the disclosure. For convenience, FIG. 11 is described with respect to FIG. 1. The operation of FIG. 11 is an operation for detecting and classifying cardiac arrhythmia in patient 4. Specifically, the operation of FIG. 11 depicts an implementation where computing system 24 uses feature delineation in series with machine learning arrhythmia detection of the machine learning system to build a dictionary of arrhythmias for use in cardiac arrhythmia detection, classification, and reporting.

The operation of FIG. 11 monitors cardiac electrogram data for patient 4, annotates detected arrhythmia, and reports such arrhythmia to a monitoring center. In some examples, the operation of FIG. 11 takes place within a centralized location such as the monitoring center. As another example, the operation of FIG. 11 may take place at a clinic on a patient-by-patient basis. As depicted in FIG. 11, IMD 10 senses cardiac electrogram data of patient 4 (1102). Computing system 24 further applies feature delineation to the cardiac electrogram data to detect one or more cardiac features (1104). The operation of steps 1102 and 1104 may occur in a substantially similar fashion to steps 502 and 504 of FIG. 5, respectively.

Computing system 24 further applies feature delineation to the cardiac electrogram data to detect one or more episodes of arrhythmia (1106). In some examples, the feature delineation causes a cardiac electrogram auto-trigger. In the example of FIG. 11, computing system 24 performs the feature delineation. However, in other examples, the arrhythmia detection and cardiac electrogram episode auto-trigger may occur on another device, such as IMD 10, external device 12, or another external medical device, or via post-processing in Holter-like systems.

If an episode of arrhythmia has been triggered from a specific patient for the first time, computing system 24 presents the episode for arrhythmia review such that the episode may be used as a reference episode in a patient-specific "episode dictionary." For example, in response to detecting an episode of arrhythmia, computing system 24 determines whether the episode of arrhythmia is the first detected episode. If the episode of arrhythmia is the first detected episode (e.g., "YES" block of 1108), computing system 24 generates a report of the episode of arrhythmia and submits the report to a monitoring center or clinician for evaluation (1110). For example, if an episode is a first AF-trigger, the episode is presented for monitoring center review. As another example, if an episode is a first AF trigger that occurs at night, the episode is presented for monitoring center review. In one example, the report includes an indication that the episode of arrhythmia has occurred in the patient and one or more of the cardiac features that coincide with the episode of arrhythmia. Computing system 24 receives, from the monitoring center, an indication verifying whether the cardiac features included in the report are indicative of an episode of arrhythmia. In an example where the cardiac features are indicative of an episode of arrhythmia, computing system 24 further receives a classification of the type of arrhythmia indicated by the cardiac features included in the report. Computing system 24 may store the indication of the classification of the type of arrhythmia together with the cardiac features in a database so as to build a "dictionary" of cardiac arrhythmia.

In some examples, computing system 24 may detect multiple episodes of arrhythmia that have similar arrhythmia content, annotations, and/or cardiac features. For example, with respect to atrial fibrillation (AF) monitoring, most episode triggers have AF. Another example is where feature delineation may generate several false triggers of arrhythmia, due to patient-specific reasons such as signal acquisition location and orientation (e.g., PACs with low-amplitude P-waves). For example, computing system 24 may input any subsequently detected episode to a machine learning model (with other episode characteristics such as trigger reason, activity level, and time of day). The machine learning model of the machine learning system compares features of the episode to features of episodes in the "episode dictionary" of patient 4. If the machine learning model determines that a similar episode is present in the dictionary with a high degree of confidence, then the original monitoring center annotations are used as-is for reporting the episode. If no similar episode is identified, then computing system 24 may determine that the episode characteristics are different and therefore present the episode for monitoring center review and reporting. Thus, the operation of FIG. 11 may increase the efficiency of arrhythmia annotation by minimizing redundant annotations in arrhythmia episodes that have similar characteristics so as to reduce the volume of arrhythmia episodes that require monitoring center review.

The techniques of the disclosure may provide the further advantage that the machine learning model of the machine learning system need not be tuned to detect a wide variety of arrhythmias. Instead, the machine learning model may be tuned only to accurately identify a new episode as similar or dissimilar to a previous episode. For example, if there is similarity between two episodes of arrhythmia, then computing system 24 may apply the previous, patient-specific findings to the new episode as well. If there is dissimilarity, then computing system 24 may request a human expert to make a determination of whether the episode is an episode of arrhythmia, and/or the type of arrhythmia presented by the episode. Accordingly, the machine learning model is not required to identify specific arrhythmias with a high level of confidence. The machine learning model needs only to be accurate in identifying differences between two episodes of arrhythmia in order to accurately present episodes with different cardiac features (e.g., novel or unclassified rhythm content) for human review. Thus, the techniques of the disclosure may allow computing system 24 to detect episodes of arrhythmia that machine learning model 150 has not been specifically trained to detect. Furthermore, the techniques of the disclosure may reduce the complexity of the machine learning model while retaining high accuracy in arrhythmia detection and classification.

For example, with respect to the operation of FIG. 11, if the episode of arrhythmia is not the first detected episode (e.g., "NO" block of 1108), the machine learning system applies a machine learning model to the detected cardiac features to compare the cardiac features to other cardiac features of previous episodes of arrhythmia (1112). For example, the machine learning system may apply the machine learning model to the detected cardiac features to determine whether the cardiac features match other cardiac features of previous episodes of arrhythmia and an estimate of a confidence level or certainty in the comparison. In some examples, computing system 24 resets the similarity comparison after a certain duration (e.g., every day) or upon demand (e.g., when patient medication changes occur). This may ensure that some episodes of arrhythmia are reviewed by the monitoring center or clinician intermittently to ensure that new or changing arrhythmias are not missed.

In response to determining that the machine learning model does not have a high confidence level or certainty in the comparison (e.g., "NO" block of 1114), computing system 24 generates a report of the episode of arrhythmia and submits the report to a monitoring center or clinician for evaluation (1110). Computing system 24 receives an indication verifying that the cardiac features included in the report are indicative of an episode of arrhythmia and a classification of the type of arrhythmia, and store the indication of the classification of the type of arrhythmia together with the cardiac features in the database so as to update the dictionary of cardiac arrhythmia with the detected cardiac features and a classification of arrhythmia indicated by the detected cardiac features.

In response to determining that the machine learning model does have a high confidence level or certainty in the comparison (e.g., "YES" block of 1114), computing system 24 may determine that the cardiac features are indicative of the type of a previous episode of arrhythmia. Computing system 24 generates a report of the arrhythmia (1116) and outputs the report to the monitoring center (1118). The operation of steps 1116 and 1118 may occur in a substantially similar fashion to steps 512 and 514 of FIG. 5, respectively.

In some examples, the techniques of the disclosure include a system that comprises means to perform any method described herein. In some examples, the techniques of the disclosure include a computer-readable medium comprising instructions that cause processing circuitry to perform any method described herein.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module, unit, or circuit for purposes of clarity, it should be understood that the techniques of this disclosure may be performed by a combination of units, modules, or circuitry associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" or "processing circuitry" as used herein may refer to any of the foregoing structure or any other physical structure suitable for implementation of the described techniques. Also, the techniques could be fully implemented in one or more circuits or logic elements.

## Claims

1. A system (2) comprising:
a medical device (10) configured to sense cardiac electrogram data of a patient; and
a computing device (400) comprising processing circuitry (402) configured to:
receive, from the medical device (10), the cardiac electrogram data of the patient for an episode of bradycardia or pause recorded by the medical device;
identify a plurality of heartbeats within the cardiac electrogram data for the episode of bradycardia or pause;
determine, based on the cardiac electrogram data, whether atrioventricular block has occurred during the episode of bradycardia or pause; and
responsive to determining that atrioventricular block has occurred during the episode of bradycardia or pause, output a report comprising:
an indication that atrioventricular block has occurred during the episode of bradycardia or pause; and
at least one of R-R intervals or P-R intervals that coincide with the atrioventricular block that has occurred during the episode of bradycardia or pause.

2. The system of claim 1, wherein the processing circuitry (402) is further configured to:
for each of the plurality of heartbeats:
determine a R-R interval;
determine whether a P-wave is detectable in the cardiac electrogram data; and
responsive to determining that the P-wave is detectable, determine a P-R interval.

3. The system of claim 2, wherein the processing circuitry (402) is further configured to, responsive to determining that one or more P-waves are not detectable in the cardiac electrogram data for the episode of bradycardia or pause, output a report comprising an indication that whether atrioventricular block has occurred during the episode of bradycardia or pause is indeterminate.

4. The system of claim 2 or 3, wherein the processing circuitry (402) is further configured to determine, for each of the plurality of heartbeats, whether the R-R interval satisfies an R-R interval threshold, wherein the processing circuitry is configured to output the report comprising P-R intervals by excluding P-R intervals for heartbeats for which the R-R interval threshold is not satisfied.

5. The system of claim 4, wherein the processing circuitry (402) is configured to output the report comprising P-R intervals by excluding P-R intervals for heartbeats that adjacently follow a respective premature ventricular contraction heartbeat.

6. The system of any of claims 1 to 4, wherein the processing circuitry (402) is further configured to, for each of the plurality of heartbeats, determine whether a R-R interval satisfies an R-R interval threshold, wherein the processing circuitry is configured to determine whether atrioventricular block has occurred during the episode of bradycardia or pause by excluding P-R intervals for heartbeats for which the R-R interval threshold is not satisfied; and/or
further comprising selecting, by the computing device, a medical device sensitivity based on user input.

7. The system of claim 6, wherein the processing circuitry (402) is configured to determine whether atrioventricular block has occurred during the episode of bradycardia or pause by excluding P-R intervals for heartbeats that adjacently follow a premature ventricular contraction heartbeat.

8. The system of any of claims 1 to 7, wherein the processing circuitry (402) is configured to determine whether atrioventricular block has occurred during the episode of bradycardia or pause by applying a machine learning system to the cardiac electrogram data.

9. The system of claim 8, wherein the processing circuitry (402) is configured to apply the machine learning system by performing feature-based delineation of the cardiac electrogram data to obtain, in addition to the R-R intervals and P-R intervals, one or more cardiac features present in the cardiac electrogram data, wherein the report further comprises the one or more cardiac features.

10. The system of claim 9, wherein the processing circuitry (402) is configured to perform feature-based delineation of the cardiac electrogram data to obtain the one or more cardiac features present in the cardiac electrogram data by performing at least one of QRS detection, refractory processing, noise processing, or delineation of the cardiac electrogram data.

11. The system of claim 9 or 10, wherein the one or more cardiac features present in the cardiac electrogram data comprise one or more of a mean heartrate of the patient, a minimum heartrate of the patient, a maximum heartrate of the patient, a variability of heartrate of the patient, one or more amplitudes of one or more features of an electrocardiogram of the patient, or an interval between the one or more features of the electrocardiogram of the patient.

12. The system of any of claims 8 to 11, wherein a machine learning model of the machine learning system is trained using cardiac electrogram data for a plurality of patients, wherein the cardiac electrogram data for the plurality of patients comprises a plurality of electrocardiogram waveforms, and wherein at least some of the electrocardiogram waveforms are labeled with atrioventricular block.

13. The system of claim 1 to 12, wherein the episode of bradycardia or pause is recorded by the medical device in response to the R-R intervals coinciding with the atrioventricular block that has occurred during the episode of bradycardia or pause satisfying a duration condition.

14. The system of claim 1 to 13, wherein determining, by the computing device, whether atrioventricular block has occurred during the episode of bradycardia or pause is based on the P-R intervals coinciding with the atrioventricular block that has occurred during the episode of bradycardia or pause.

15. A non-transitory computer-readable storage medium having instructions stored thereon that, when executed, cause processing circuitry (402) of a computing device (400) to:
receive, from a medical device (10), cardiac electrogram data of the patient for an episode of bradycardia or pause recorded by the medical device (10);
identify a plurality of heartbeats within the cardiac electrogram data for the episode of bradycardia or pause;
determine, based on the cardiac electrogram data, whether atrioventricular block has occurred during the episode of bradycardia or pause; and
responsive to determining that atrioventricular block has occurred during the episode of bradycardia or pause, output a report comprising:
an indication that atrioventricular block has occurred during the episode of bradycardia or pause; and
at least one of the R-R intervals or the P-R intervals that coincide with the atrioventricular block that has occurred during the episode of bradycardia or pause.

## Patentansprüche

1. System (2), umfassend:
eine medizinische Vorrichtung (10), die dazu ausgelegt ist, Herzelektrogrammdaten eines Patienten zu erfassen; und
eine Rechenvorrichtung (400), umfassend eine Verarbeitungsschaltung (402), die hierzu ausgelegt ist:
Empfangen, von der medizinischen Vorrichtung (10), der Herzelektrogrammdaten des Patienten für eine Episode von Bradykardie oder eine Pause, die durch die medizinische Vorrichtung aufgezeichnet werden;
Identifizieren einer Mehrzahl von Herzschlägen in den Herzelektrogrammdaten für die Episode von Bradykardie oder die Pause;
Bestimmen, basierend auf den Herzelektrogrammdaten, ob ein atrioventrikulärer Block während der Episode von Bradykardie oder der Pause aufgetreten ist; und
in Reaktion auf das Bestimmen, dass ein atrioventrikulärer Block während der Episode von Bradykardie oder der Pause aufgetreten ist, Ausgeben eines Berichts, der dies umfasst:
eine Angabe, dass ein atrioventrikulärer Block während der Episode von Bradykardie oder der Pause aufgetreten ist; und
wenigstens eines von R-R-Intervallen oder P-R-Intervallen, die mit dem atrioventrikulären Block zusammenfallen, der während der Episode von Bradykardie oder der Pause aufgetreten ist.

2. System nach Anspruch 1, wobei die Verarbeitungsschaltung (402) ferner hierzu ausgelegt ist:
für jedes der Vielzahl von Bildern:
Bestimmen eines R-R-Intervalls;
Bestimmen, ob eine P-Welle in den Herzelektrogrammdaten detektierbar ist; und
in Reaktion auf das Bestimmen, dass die P-Welle detektierbar ist, Bestimmen eines P-R-Intervalls.

3. System nach Anspruch 2, wobei die Verarbeitungsschaltung (402) ferner dazu ausgelegt ist, in Reaktion auf das Bestimmen, dass ein oder mehrere P-Wellen in dem Herzelektrogrammdaten für die Episode von Bradykardie oder die Pause nicht detektierbar sind, einen Bericht auszugeben, der eine Angabe umfasst, dass unbestimmt ist, ob ein atrioventrikulärer Block während der Episode von Bradykardie oder der Pause aufgetreten ist.

4. System nach Anspruch 2 oder 3, wobei die Verarbeitungsschaltung (402) ferner dazu ausgelegt ist, für jeden der Mehrzahl von Herzschlägen zu bestimmen, ob das R-R-Intervall einen R-R-Intervallschwellwert erfüllt, wobei die Verarbeitungsschaltung dazu ausgelegt ist, den P-R-Intervalle umfassenden Bericht auszugeben, indem P-R-Intervalle für Herzschläge, bei denen der R-R-Intervallschwellwert nicht erfüllt wird, ausgeschlossen werden.

5. System nach Anspruch 4, wobei die Verarbeitungsschaltung (402) dazu ausgelegt ist, den P-R-Intervalle umfassenden Bericht auszugeben, indem P-R-Intervalle für Herzschläge, die direkt auf einen jeweiligen vorzeitigen ventrikulären Kontraktionsherzschlag folgen, ausgeschlossen werden.

6. System nach einem der Ansprüche 1 bis 4, wobei die Verarbeitungsschaltung (402) ferner dazu ausgelegt ist, für jeden der Mehrzahl von Herzschlägen zu bestimmen, ob ein R-R-Intervall einen R-R-Intervallschwellwert erfüllt, wobei die Verarbeitungsschaltung dazu ausgelegt ist zu bestimmen, ob ein atrioventrikulärer Block während der Episode von Bradykardie oder der Pause aufgetreten ist, indem P-R-Intervalle für Herzschläge, bei denen der R-R-Intervallschwellwert nicht erfüllt wird, ausgeschlossen werden; und/oder
ferner umfassend das Auswählen, durch die Rechenvorrichtung, einer Empfindlichkeit der medizinischen Vorrichtung basierend auf einer Benutzereingabe.

7. System nach Anspruch 6, wobei die Verarbeitungsschaltung (402) dazu ausgelegt ist zu bestimmen, ob ein atrioventrikulärer Block während der Episode von Bradykardie oder der Pause aufgetreten ist, indem P-R-Intervalle für Herzschläge, die direkt auf einen vorzeitigen ventrikulären Kontraktionsherzschlag folgen, ausgeschlossen werden.

8. System nach einem der Ansprüche 1 bis 7, wobei die Verarbeitungsschaltung (402) dazu ausgelegt ist zu bestimmen, ob ein atrioventrikulärer Block während der Episode von Bradykardie der Pause aufgetreten ist, indem ein maschinelles Lernsystem auf die Herzelektrogrammdaten angewendet wird.

9. System nach Anspruch 8, wobei die Verarbeitungsschaltung (402) dazu ausgelegt ist, das maschinelle Lernsystem anzuwenden, indem eine merkmalsbasierte Abgrenzung der Herzelektrogrammdaten durchgeführt wird, um, zusätzlich zu den R-R-Intervallen und den P-R-Intervallen, ein oder mehrere Herzmerkmale zu erhalten, die in den Herzelektrogrammdaten vorliegen, wobei der Bericht ferner die ein oder mehreren Herzmerkmale umfasst.

10. System nach Anspruch 9, wobei die Verarbeitungsschaltung (402) dazu ausgelegt ist, eine merkmalsbasierte Abgrenzung der Herzelektrogrammdaten durchzuführen, um die ein oder mehreren Herzmerkmale zu erhalten, die in den Herzelektrogrammdaten vorliegen, indem wenigstens eines von einer QRS-Detektion, einer refraktorischen Verarbeitung, einer Rauschverarbeitung oder einer Abgrenzung der Herzelektrogrammdaten durchgeführt wird.

11. System nach Anspruch 9 oder 10, wobei die ein oder mehreren Herzmerkmale, die in den Herzelektrogrammdaten vorliegen, eines oder mehrere hiervon umfassen: eine mittlere Herzfrequenz des Patienten, eine minimale Herzfrequenz des Patienten, eine maximale Herzfrequenz des Patienten, eine Variabilität der Herzfrequenz des Patienten, ein oder mehrere Amplituden eines oder mehrerer Merkmale eines Elektrokardiogramms des Patienten oder ein Intervall zwischen den ein oder mehreren Merkmalen des Elektrokardiogramms des Patienten.

12. System nach einem der Ansprüche 8 bis 11, wobei ein maschinelles Lernmodell des maschinellen Lernsystems unter Verwendung von Herzelektrogrammdaten für eine Mehrzahl von Patienten trainiert wird, wobei die Herzelektrogrammdaten für die Mehrzahl von Patienten eine Mehrzahl von Elektrokardiogramm-Wellenformen umfasst und wobei wenigstens einige der Elektrokardiogramm-Wellenformen mit atrioventrikulärer Block beschriftet sind.

13. System nach Anspruch 1 bis 12, wobei die Episode von Bradykardie oder die Pause durch die medizinische Vorrichtung in Reaktion darauf aufgezeichnet werden, dass die R-R-Intervalle mit dem atrioventrikulären Block zusammenfallen, der aufgetreten ist, während die Episode von Bradykardie oder die Pause eine Dauerbedingung erfüllen.

14. System nach Anspruch 1 bis 13, wobei das Bestimmen, durch die Rechenvorrichtung, ob ein atrioventrikulärer Block während der Episode von Bradykardie oder der Pause aufgetreten ist, darauf basiert, dass die P-R-Intervalle mit dem atrioventrikulären Block zusammenfallen, der während der Episode von Bradykardie oder der Pause aufgetreten ist.

15. Nicht-transitorisches computerlesbares Datenspeichermedium mit darauf gespeicherten Anweisungen, die bei Ausführung die Verarbeitungsschaltung (402) einer Rechenvorrichtung (400) hierzu veranlassen:
Empfangen, von einer medizinischen Vorrichtung (10), von Herzelektrogrammdaten des Patienten für eine Episode von Bradykardie oder eine Pause, die durch die medizinische Vorrichtung (10) aufgezeichnet werden;
Identifizieren einer Mehrzahl von Herzschlägen in den Herzelektrogrammdaten für die Episode von Bradykardie oder die Pause;
Bestimmen, basierend auf den Herzelektrogrammdaten, ob ein atrioventrikulärer Block während der Episode von Bradykardie oder der Pause aufgetreten ist; und
in Reaktion auf das Bestimmen, dass ein atrioventrikulärer Block während der Episode von Bradykardie oder der Pause aufgetreten ist, Ausgeben eines Berichts, der dies umfasst:
eine Angabe, dass ein atrioventrikulärer Block während der Episode von Bradykardie oder der Pause aufgetreten ist; und
wenigstens eines von den R-R-Intervallen oder den P-R-Intervallen, die mit dem atrioventrikulären Block zusammenfallen, der während der Episode von Bradykardie oder der Pause aufgetreten ist.

## Revendications

1. Système (2) comprenant :
un dispositif médical (10) configuré pour détecter des données d'électrogramme cardiaque d'un patient ; et
un dispositif informatique (400) comprenant un circuit de traitement (402) configuré pour :
recevoir, à partir du dispositif médical (10), les données d'électrogramme cardiaque du patient pour un épisode de bradycardie ou de pause enregistré par le dispositif médical ;
identifier une pluralité de battements cardiaques dans les données d'électrogramme cardiaque pour l'épisode de bradycardie ou de pause ;
déterminer, sur la base des données d'électrogramme cardiaque, si un bloc auriculo-ventriculaire s'est produit pendant l'épisode de bradycardie ou de pause ; et
en réponse à la détermination de la survenue d'un bloc auriculo-ventriculaire pendant l'épisode de bradycardie ou de pause, produire un rapport comprenant :
une indication que le bloc auriculo-ventriculaire est survenu pendant l'épisode de bradycardie ou de pause ; et
au moins l'un des intervalles R-R ou P-R coïncidant avec le bloc auriculo-ventriculaire survenu pendant l'épisode de bradycardie ou de pause.

2. Système selon la revendication 1, le circuit de traitement (402) étant en outre configuré pour :
pour chacun de la pluralité de battements cardiaques :
déterminer un intervalle R-R ;
déterminer si une onde P est détectable dans les données d'électrogramme cardiaque ; et
en réponse à la détermination que l'onde P est détectable, déterminer un intervalle P-R.

3. Système selon la revendication 2, le circuit de traitement (402) étant en outre configuré pour, en réponse à la détermination qu'une ou plusieurs ondes P ne sont pas détectables dans les données d'électrogramme cardiaque pour l'épisode de bradycardie ou de pause, produire un rapport comprenant une indication que si un bloc auriculo-ventriculaire s'est produit pendant l'épisode de bradycardie ou de pause est indéterminé.

4. Système selon la revendication 2 ou 3, le circuit de traitement (402) étant en outre configuré pour déterminer, pour chacun de la pluralité de battements cardiaques, si l'intervalle R-R satisfait à un seuil d'intervalle R-R, le circuit de traitement étant configuré pour produire le rapport comprenant des intervalles P-R en excluant des intervalles P-R pour des battements cardiaques pour lesquels le seuil d'intervalle R-R n'est pas satisfait.

5. Système selon la revendication 4, le circuit de traitement (402) étant configuré pour produire le rapport comprenant des intervalles P-R en excluant des intervalles P-R pour des battements cardiaques qui suivent de façon adjacente un battement cardiaque respectif de contraction ventriculaire prématurée.

6. Système selon l'une quelconque des revendications 1 à 4, le circuit de traitement (402) étant en outre configuré pour, pour chacun de la pluralité de battements cardiaques, déterminer si un intervalle R-R satisfait à un seuil d'intervalle R-R, le circuit de traitement étant configuré pour déterminer si un bloc auriculo-ventriculaire s'est produit pendant l'épisode de bradycardie ou de pause en excluant des intervalles P-R pour des battements cardiaques pour lesquels le seuil d'intervalle R-R n'est pas satisfait ; et/ou
comprenant en outre la sélection, par le dispositif informatique, d'une sensibilité de dispositif médical sur la base d'une entrée utilisateur.

7. Système selon la revendication 6, le circuit de traitement (402) étant configuré pour déterminer si un bloc auriculo-ventriculaire s'est produit pendant l'épisode de bradycardie ou de pause en excluant des intervalles P-R pour des battements cardiaques qui suivent de façon adjacente un battement cardiaque de contraction ventriculaire prématurée.

8. Système selon l'une quelconque des revendications 1 à 7, le circuit de traitement (402) étant configuré pour déterminer si un bloc auriculo-ventriculaire s'est produit pendant l'épisode de bradycardie ou de pause en appliquant un système d'apprentissage automatique aux données d'électrogramme cardiaque.

9. Système selon la revendication 8, le circuit de traitement (402) étant configuré pour appliquer le système d'apprentissage automatique en effectuant une délimitation basée sur des caractéristiques des données d'électrogramme cardiaque pour obtenir, en plus des intervalles R-R et des intervalles P-R, une ou plusieurs caractéristiques cardiaques présentes dans les données d'électrogramme cardiaque, le rapport comprenant en outre la ou les caractéristiques cardiaques.

10. Système selon la revendication 9, le circuit de traitement (402) étant configuré pour effectuer une délimitation basée sur des caractéristiques des données d'électrogramme cardiaque pour obtenir la ou les caractéristiques cardiaques présentes dans les données d'électrogramme cardiaque en effectuant au moins l'une parmi la détection QRS, le traitement réfractaire, le traitement de bruit ou la délimitation des données d'électrogramme cardiaque.

11. Système selon la revendication 9 ou 10, la ou les caractéristiques cardiaques présentes dans les données d'électrogramme cardiaque comprenant une ou plusieurs parmi un rythme cardiaque moyen du patient, un rythme cardiaque minimum du patient, un rythme cardiaque maximum du patient, une variabilité du rythme cardiaque du patient, une ou plusieurs amplitudes d'une ou plusieurs caractéristiques d'un électrocardiogramme du patient, ou un intervalle entre la ou les caractéristiques de l'électrocardiogramme du patient.

12. Système selon l'une quelconque des revendications 8 à 11, un modèle d'apprentissage automatique du système d'apprentissage automatique étant entraîné en utilisant des données d'électrogramme cardiaque pour une pluralité de patients, les données d'électrogramme cardiaque pour la pluralité de patients comprenant une pluralité de formes d'onde d'électrocardiogramme, et au moins certaines des formes d'onde d'électrocardiogramme étant marquées avec un bloc auriculo-ventriculaire.

13. Système selon les revendications 1 à 12, l'épisode de bradycardie ou de pause étant enregistré par le dispositif médical en réponse aux intervalles R-R coïncidant avec le bloc auriculo-ventriculaire qui s'est produit pendant l'épisode de bradycardie ou de pause satisfaisant à une condition de durée.

14. Système selon les revendications 1 à 13, la détermination, par le dispositif informatique, si un bloc auriculo-ventriculaire s'est produit pendant l'épisode de bradycardie ou de pause étant basée sur les intervalles P-R coïncidant avec le bloc auriculo-ventriculaire qui s'est produit pendant l'épisode de bradycardie ou de pause.

15. Support de stockage non transitoire lisible par ordinateur sur lequel sont stockées des instructions qui, lorsqu'elles sont exécutées, amènent le circuit de traitement (402) d'un dispositif informatique (400) à :
recevoir, à partir d'un dispositif médical (10), des données d'électrogramme cardiaque du patient pour un épisode de bradycardie ou de pause enregistrée par le dispositif médical (10) ;
identifier une pluralité de battements cardiaques dans les données d'électrogramme cardiaque pour l'épisode de bradycardie ou de pause ;
déterminer, sur la base des données d'électrogramme cardiaque, si un bloc auriculo-ventriculaire s'est produit pendant l'épisode de bradycardie ou de pause ; et
en réponse à la détermination de la survenue d'un bloc auriculo-ventriculaire pendant l'épisode de bradycardie ou de pause, produire un rapport comprenant :
une indication que le bloc auriculo-ventriculaire est survenu pendant l'épisode de bradycardie ou de pause ; et
au moins un des intervalles R-R ou des intervalles P-R qui coïncident avec le bloc auriculo-ventriculaire qui s'est produit pendant l'épisode de bradycardie ou de pause.
